# EUROPEAN PATENT SPECIFICATION

(11) **EP 2 661 256 B1**
(45) Date of publication and mention of the grant of the patent: **14.11.2018**
(21) Application number: 12700583.3
(22) Date of filing: 03.01.2012
(51) Int. Cl.: A61K 9/16, A61K 9/20, A61K 31/335, A61P 3/10

(54) **COMPOSITIONS COMPRISING (2S,3R,4R,5S,6R)-2-(4- CHLORO-3-(4-ETHOXYBENZYL)PHENYL)-6-(METHYLTHIO)TETRAHYDRO-2H-PYRAN-3,4,5-TRIOL**
ZUSAMMENSETZUNGEN MIT (2S,3R,4R,5S,6R)-2-(4- CHLOR-3-(4-ETHOXYBENZYL)PHENYL)-6-(METHYLTHIO)TETRAHYDRO-2H-PYRAN-3,4,5-TRIOL
COMPOSITIONS CONTENANT DE (2S,3R,4R,5S,6R)-2-(4- CHLORO-3-(4-ÉTHOXYBENZYL)PHÉNYL)-6-(MÉTHYLTHIO)TÉTRAHYDRO-2H-PYRAN-3,4,5-TRIOL

(30) Priority: 05.01.2011 US 201161430027 P
(43) Date of publication of application: 13.11.2013
(73) Proprietor: Lexicon Pharmaceuticals, Inc., The Woodlands, TX 77381 (US)
(72) Inventor: CHEN, Jinling, Houston Texas 77059 (US); NYAMWEYA, Nasser, N., Nairobi (KE); ONG, Kenneth, K. H., Scarborough Ontario M1S 2W3 (CA)
(74) Representative: Abel & Imray
(86) International application number: PCT/US2012/020042
(87) International publication number: WO 2012/094293

(56) References cited:
- WO-A1-2011/109333
- WO-A2-2011/060256
- US-A1- 2006 009 400
- US-A1- 2010 016 422
- US-A1- 2011 077 212
- US-B1- 6 414 126
- WASHBURN WILLIAM N: "Evolution of sodium glucose co-transporter 2 inhibitors as anti-diabetic agents", EXPERT OPINION ON THERAPEUTIC PATENTS, INFORMA HEALTHCARE, GB, vol. 19, no. 11, 1 November 2009 (2009-11-01), pages 1485-1499, XP002593697, ISSN: 1354-3776, DOI: 10.1517/13543770903337828
- FREIMAN J ET AL: "LX4211, an SGLT2 Inhibitor, Shows Improvements in Cardiovascular Risk Factors over 4 Weeks in Patients with Type 2 Diabetes Mellitus", ENDOCRINE REVIEWS , vol. 31, no. 3 June 2010 (2010-06), page 2237, XP002632760, Retrieved from the Internet: URL:http://www.posters2view.com/endo/ [retrieved on 2011-04-04]

## Description

This application claims priority to U.S. provisional patent application no. 61/430,027, filed January 5, 2011.

### 1. FIELD OF THE INVENTION

This invention relates to methods of improving the cardiovascular and/or metabolic health of patients, particularly those suffering from type 2 diabetes, and to compounds and pharmaceutical compositions useful therein.

### 2. BACKGROUND

Type 2 diabetes mellitus (T2DM) is a disorder characterized by elevated serum glucose. One way of reducing serum glucose in patients suffering from the disease is by inhibiting glucose reabsorption in the kidney. The kidney plays an important role in the overall control of glucose, since glucose is filtered through the glomeruli at the rate of approximately 8 g/h and is almost completely reabsorbed in the proximal tubule via sodium-glucose cotransporters (SGLTs). Komoroski, B., et al., Clin Pharmacol Ther. 85(5):513-9 (2009). Sodium-glucose cotransporter 2 (SGLT2) is one of 14 transmembrane-domain SGLTs, and is responsible for reabsorbing most of the glucose filtered at the glomerulus. Thus, inhibition of SGLT2 is a rational approach to treating T2DM. *Id*.

A large number of SGLT2 inhibitors have been reported. *See, e.g.,* U.S. patent nos. 6,414,126; 6,555,519; and 7,393,836. One of them, dapagliflozin, has been administered to T2DM patients with promising results. In particular, patients randomized to the compound in a 14-day study exhibited reduced fasting plasma levels and improved glucose tolerance compared to placebo. Komoroski at 513. In a 12-week study, patients randomized to the compound exhibited an improvement in hemoglobin A1c, some weight loss, and some improvement in systolic blood pressure compared to placebo. List, J.F., et al., Diabetes Care. 32(4):650-7 (2009).

Most pharmaceutical efforts directed at discovering and developing inhibitors of SGLT2 "have focused on devising inhibitors selective for the SGLT2 transporter." Washburn, W.N., Expert Opin. Ther. Patents 19(11):1485,1499, 1486 (2009). This is apparently based, at least in part, on the fact that while humans lacking a functional SGLT2 gene appear to live normal lives-apart from exhibiting high urinary glucose excretion-those bearing a SGLT1 gene mutation experience glucose-galactose malsorption. *Id.* Unlike SGLT2, which is expressed exclusively in the human kidney, SGLT1 is also expressed in the small intestine and heart. *Id.*

### 3. SUMMARY OF THE INVENTION

This invention is directed, in part, to a tablet comprising an API, croscarmellose sodium, silicon dioxide and microcrystalline cellulose, wherein the active pharmaceutical ingredient ("API") is crystalline anhydrous (2S,3R,4R,5S,6R)-2-(4-chloro-3-(4-ethoxybenzyl)phenyl)-6-(methylthio)tetrahydro-2H-pyran-3,4,5-triol.

This invention is directed, in part, to a granule comprising an API, croscarmellose sodium, collodial silicon dioxide, and microcrystalline cellulose, wherein the API is (2S,3R,4R,5S,6R)-2-(4-chloro-3-(4-ethoxybenzyl)phenyl)-6-(methylthio)tetrahydro-2H-pyran-3,4,5-triol.

### 4. BRIEF DESCRIPTION OF THE FIGURES

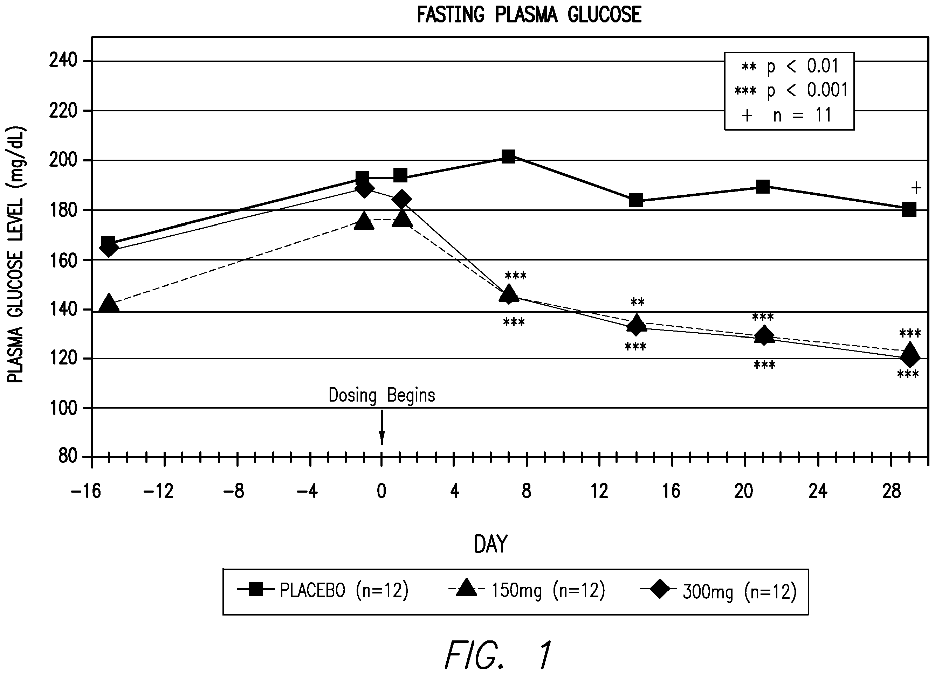

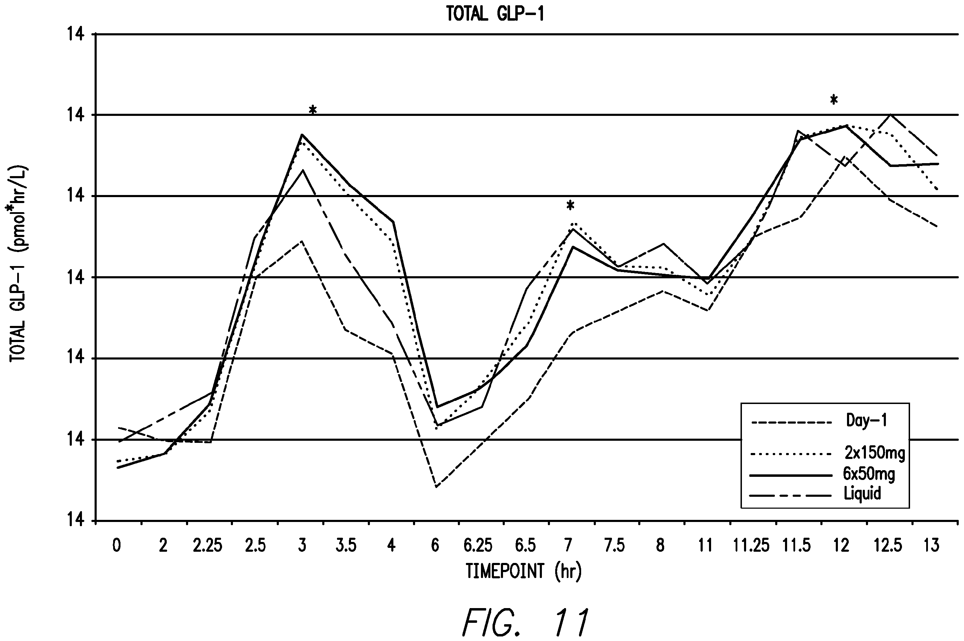

Certain aspects of this invention may be understood with reference to the figures. Figures 1-10 show results obtained from a randomized, double-blind, placebo controlled Phase 2a clinical trial, wherein 150 mg and 300 mg doses of (2S,3R,4R,5S,6R)-2-(4-chloro-3-(4-ethoxybenzyl)phenyl)-6-(methylthio)tetrahydro-2H-pyran-3,4,5-triol were orally administered in solution once daily to patients with type 2 diabetes mellitus. Figure 11 provides results obtained from a Phase 1 clinical trial, wherein both solid and liquid oral dosage forms of the compound were administered to patients with type 2 diabetes mellitus.
FIG. 1 shows the plasma glucose levels of patients in the placebo group and in the 150 mg/day and 300 mg/day treatment groups over the course of the Phase 2a study.
FIG. 2 shows each group's mean results in a glucose tolerance test administered over the course of the study.
FIG. 3 shows each group's mean glucose plasma level area under the curve (AUC) over the course of the study.
FIG. 4 shows the results of each group's mean homeostatic model assessment (HOMA) value. Measurements were obtained before the study began and again on day 27.
FIG. 5 provides measurements of each group's mean post-prandial glucose level over the course of the study.
FIG. 6 provides measurements of each group's mean plasma fructosamine level over the course of the study.
FIG. 7 provides each group's mean percent change in hemoglobin A1c level over the course of the study.
FIG. 8 shows the change in each group's mean diastolic blood pressure as measured on day 28 of the study compared to baseline.
FIG. 9 shows the change in each group's mean systolic blood pressure as measured on day 28 of the study compared to baseline.
FIG. 10 shows the change in each group's mean arterial pressure as measured on day 28 of the study compared to baseline.
FIG. 11 shows the effects of a single dose of one of two solid formulations (6x50 mg tablets or 2x150 mg tablets) and a liquid formulation of (2S,3R,4R,5S,6R)-2-(4-chloro-3-(4-ethoxybenzyl)phenyl)-6-(methylthio)tetrahydro-2H-pyran-3,4,5-triol on the total GLP-1 levels of patients with type 2 diabetes mellitus, as determined in a Phase 1 study.

### 5. DETAILED DESCRIPTION

This invention is based, in part, on findings obtained from a randomized, double-blind, placebo controlled Phase 2a clinical trial, wherein 150 mg/day and 300 mg/day doses of a compound of the invention were orally administered in a liquid to patients with type 2 diabetes mellitus. The compound was (2S,3R,4R,5S,6R)-2-(4-chloro-3-(4-ethoxybenzyl)phenyl)-6-(methylthio)tetrahydro-2H-pyran-3,4,5-triol, which has the structure:

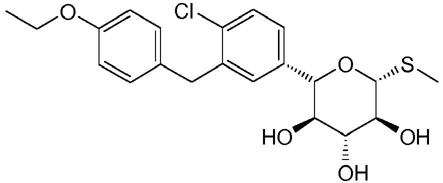

This invention is further based on findings obtained from a randomized, double-blind, placebo controlled Phase 1 clinical trial that compared liquid and solid dosage forms of the compound.

### 5.1. Definitions

Unless otherwise indicated, the term "about," when used in association with a numerical value, means the value should be considered as including the error (e.g., standard error) associated with obtaining or deriving it.

Unless otherwise indicated, the term "alkenyl" means a straight chain, branched and/or cyclic hydrocarbon having from 2 to 20 (e.g., 2 to 10 or 2 to 6) carbon atoms, and including at least one carbon-carbon double bond. Representative alkenyl moieties include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl and 3-decenyl.

Unless otherwise indicated, the term "alkoxy" means an -O-alkyl group. Examples of alkoxy groups include, but are not limited to, -OCH₃, -OCH₂CH₃, -O(CH₂)₂CH₃, -O(CH₂)₃CH₃, -O(CH₂)₄CH₃, and -O(CH₂)₅CH₃.

Unless otherwise indicated, the term "alkyl" means a straight chain, branched and/or cyclic ("cycloalkyl") hydrocarbon having from 1 to 20 (e.g., 1 to 10 or 1 to 4) carbon atoms. Alkyl moieties having from 1 to 4 carbons are referred to as "lower alkyl." Examples of alkyl groups include, but are not limited to, methyl, ethyl, propyl, isopropyl, n-butyl, t-butyl, isobutyl, pentyl, hexyl, isohexyl, heptyl, 4,4-dimethylpentyl,°Ctyl, 2,2,4-trimethylpentyl, nonyl, decyl, undecyl and dodecyl. Cycloalkyl moieties may be monocyclic or multicyclic, and examples include cyclopropyl, cyclobutyl, cyclopentyl, cyclohexyl, and adamantyl. Additional examples of alkyl moieties have linear, branched and/or cyclic portions (*e*.*g*., 1-ethyl-4-methyl-cyclohexyl). The term "alkyl" includes saturated hydrocarbons as well as alkenyl and alkynyl moieties.

Unless otherwise indicated, the term "alkylaryl" or "alkyl-aryl" means an alkyl moiety bound to an aryl moiety.

Unless otherwise indicated, the term "alkylheteroaryl" or "alkyl-heteroaryl" means an alkyl moiety bound to a heteroaryl moiety.

Unless otherwise indicated, the term "alkylheterocycle" or "alkyl-heterocycle" means an alkyl moiety bound to a heterocycle moiety.

Unless otherwise indicated, the term "alkynyl" means a straight chain, branched or cyclic hydrocarbon having from 2 to 20 (*e*.*g*., 2 to 20 or 2 to 6) carbon atoms, and including at least one carbon-carbon triple bond. Representative alkynyl moieties include acetylenyl, propynyl, 1-butynyl, 2-butynyl, 1-pentynyl, 2-pentynyl, 3-methyl-1-butynyl, 4-pentynyl, 1-hexynyl, 2-hexynyl, 5-hexynyl, 1-heptynyl, 2-heptynyl, 6-heptynyl, 1-octynyl, 2-octynyl, 7-octynyl, 1-nonynyl, 2-nonynyl, 8-nonynyl, 1-decynyl, 2-decynyl and 9-decynyl.

Unless otherwise indicated, the term "aryl" means an aromatic ring or an aromatic or partially aromatic ring system composed of carbon and hydrogen atoms. An aryl moiety may comprise multiple rings bound or fused together. Examples of aryl moieties include, but are not limited to, anthracenyl, azulenyl, biphenyl, fluorenyl, indan, indenyl, naphthyl, phenanthrenyl, phenyl, 1,2,3,4-tetrahydro-naphthalene, and tolyl.

Unless otherwise indicated, the term "arylalkyl" or "aryl-alkyl" means an aryl moiety bound to an alkyl moiety.

Unless otherwise indicated, the term "dual SGLT1/2 inhibitor" refers to a compound having a ratio of SGLT1 IC₅₀ to SGLT2 IC₅₀ of less than about 75, 50, or 25.

Unless otherwise indicated, the terms "halogen" and "halo" encompass fluorine, chlorine, bromine, and iodine.

Unless otherwise indicated, the term "heteroalkyl" refers to an alkyl moiety (e.g., linear, branched or cyclic) in which at least one of its carbon atoms has been replaced with a heteroatom (*e*.*g*., N, O or S).

Unless otherwise indicated, the term "heteroaryl" means an aryl moiety wherein at least one of its carbon atoms has been replaced with a heteroatom (*e*.*g*., N, O or S). Examples include, but are not limited to, acridinyl, benzimidazolyl, benzofuranyl, benzoisothiazolyl, benzoisoxazolyl, benzoquinazolinyl, benzothiazolyl, benzoxazolyl, furyl, imidazolyl, indolyl, isothiazolyl, isoxazolyl, oxadiazolyl, oxazolyl, phthalazinyl, pyrazinyl, pyrazolyl, pyridazinyl, pyridyl, pyrimidinyl, pyrimidyl, pyrrolyl, quinazolinyl, quinolinyl, tetrazolyl, thiazolyl, and triazinyl.

Unless otherwise indicated, the term "heteroarylalkyl" or "heteroaryl-alkyl" means a heteroaryl moiety bound to an alkyl moiety.

Unless otherwise indicated, the term "heterocycle" refers to an aromatic, partially aromatic or non-aromatic monocyclic or polycyclic ring or ring system comprised of carbon, hydrogen and at least one heteroatom (*e*.*g*., N, O or S). A heterocycle may comprise multiple (*i*.*e*., two or more) rings fused or bound together. Heterocycles include heteroaryls. Examples include, but are not limited to, benzo[1,3]dioxolyl, 2,3-dihydro-benzo[1,4]dioxinyl, cinnolinyl, furanyl, hydantoinyl, morpholinyl, oxetanyl, oxiranyl, piperazinyl, piperidinyl, pyrrolidinonyl, pyrrolidinyl, tetrahydrofuranyl, tetrahydropyranyl, tetrahydropyridinyl, tetrahydropyrimidinyl, tetrahydrothiophenyl, tetrahydrothiopyranyl and valerolactamyl.

Unless otherwise indicated, the term "heterocyclealkyl" or "heterocycle-alkyl" refers to a heterocycle moiety bound to an alkyl moiety.

Unless otherwise indicated, the term "heterocycloalkyl" refers to a non-aromatic heterocycle.

Unless otherwise indicated, the term "heterocycloalkylalkyl" or "heterocycloalkyl-alkyl" refers to a heterocycloalkyl moiety bound to an alkyl moiety.

Unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

Unless otherwise indicated, the term "pharmaceutically acceptable salts" refers to salts prepared from pharmaceutically acceptable non-toxic acids or bases including inorganic acids and bases and organic acids and bases. Suitable pharmaceutically acceptable base addition salts include, but are not limited to, metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts. Others are well-known in the art. *See, e*.*g*., Remington's Pharmaceutical Sciences, 18th ed. (Mack Publishing, Easton PA: 1990) and Remington: The Science and Practice of Pharmacy, 19th ed. (Mack Publishing, Easton PA: 1995).

Unless otherwise indicated, the terms "prevent," "preventing" and "prevention" contemplate an action that occurs before a patient begins to suffer from the specified disease or disorder, which inhibits or reduces the severity of the disease or disorder. In other words, the terms encompass prophylaxis.

Unless otherwise indicated, a "prophylactically effective amount" of a compound is an amount sufficient to prevent a disease or condition, or one or more symptoms associated with the disease or condition, or prevent its recurrence. A "prophylactically effective amount" of a compound means an amount of therapeutic agent, alone or in combination with other agents, which provides a prophylactic benefit in the prevention of the disease. The term "prophylactically effective amount" can encompass an amount that improves overall prophylaxis or enhances the prophylactic efficacy of another prophylactic agent.

Unless otherwise indicated, the term "SGLT1 IC₅₀" is the IC₅₀ of a compound determined using the *in vitro* human SGLT1 inhibition assay described in the Examples, below.

Unless otherwise indicated, the term "SGLT2 IC₅₀" is the IC₅₀ of a compound determined using the *in vitro* human SGLT2 inhibition assay described in the Examples, below.

Unless otherwise indicated, the term "substituted," when used to describe a chemical structure or moiety, refers to a derivative of that structure or moiety wherein one or more of its hydrogen atoms is substituted with an atom, chemical moiety or functional group such as, but not limited to, alcohol, aldehylde, alkoxy, alkanoyloxy, alkoxycarbonyl, alkenyl, alkyl (e.g., methyl, ethyl, propyl, t-butyl), alkynyl, alkylcarbonyloxy (-OC(O)alkyl), amide (-C(O)NH-alkyl- or-alkylNHC(O)alkyl), amidinyl (-C(NH)NH-alkyl or -C(NR)NH₂), amine (primary, secondary and tertiary such as alkylamino, arylamino, arylalkylamino), aroyl, aryl, aryloxy, azo, carbamoyl (-NHC(O)O-alkyl- or-OC(O)NH-alkyl), carbamyl (*e*.*g*., CONH₂, as well as CONH-alkyl, CONH-aryl, and CONH-arylalkyl), carbonyl, carboxyl, carboxylic acid, carboxylic acid anhydride, carboxylic acid chloride, cyano, ester, epoxide, ether (*e*.*g*., methoxy, ethoxy), guanidino, halo, haloalkyl (*e*.*g*., -CCl₃, -CF₃, -C(CF₃)₃), heteroalkyl, hemiacetal, imine (primary and secondary), isocyanate, isothiocyanate, ketone, nitrile, nitro, oxygen (*i*.*e*., to provide an oxo group), phosphodiester, sulfide, sulfonamido (*e*.*g*., SO₂NH₂), sulfone, sulfonyl (including alkylsulfonyl, arylsulfonyl and arylalkylsulfonyl), sulfoxide, thiol (*e*.*g*., sulfhydryl, thioether) and urea (-NHCONH-alkyl-). In a particular embodiment, the term substituted refers to a derivative of that structure or moiety wherein one or more of its hydrogen atoms is substituted with alcohol, alkoxy, alkyl (*e*.*g*., methyl, ethyl, propyl, t-butyl), amide (-C(O)NH-alkyl- or-alkylNHC(O)alkyl), amidinyl (-C(NH)NH-alkyl or -C(NR)NH₂), amine (primary, secondary and tertiary such as alkylamino, arylamino, arylalkylamino), aryl, carbamoyl (-NHC(O)O-alkyl- or -OC(O)NH-alkyl), carbamyl (*e*.*g*., CONH₂, as well as CONH-alkyl, CONH-aryl, and CONH-arylalkyl), halo, haloalkyl (*e*.*g*., -CCl₃, -CF₃, -C(CF₃)₃), heteroalkyl, imine (primary and secondary), isocyanate, isothiocyanate, thiol (*e*.*g*., sulfhydryl, thioether) or urea (-NHCONH-alkyl-).

Unless otherwise indicated, a "therapeutically effective amount" of a compound is an amount sufficient to provide a therapeutic benefit in the treatment or management of a disease or condition, or to delay or minimize one or more symptoms associated with the disease or condition. A "therapeutically effective amount" of a compound means an amount of therapeutic agent, alone or in combination with other therapies, which provides a therapeutic benefit in the treatment or management of the disease or condition. The term "therapeutically effective amount" can encompass an amount that improves overall therapy, reduces or avoids symptoms or causes of a disease or condition, or enhances the therapeutic efficacy of another therapeutic agent.

Unless otherwise indicated, the terms "treat," "treating" and "treatment" contemplate an action that occurs while a patient is suffering from the specified disease or disorder, which reduces the severity of the disease or disorder, or retards or slows the progression of the disease or disorder.

Unless otherwise indicated, the term "include" has the same meaning as "include, but are not limited to," and the term "includes" has the same meaning as "includes, but is not limited to." Similarly, the term "such as" has the same meaning as the term "such as, but not limited to."

Unless otherwise indicated, one or more adjectives immediately preceding a series of nouns is to be construed as applying to each of the nouns. For example, the phrase "optionally substituted alky, aryl, or heteroaryl" has the same meaning as "optionally substituted alky, optionally substituted aryl, or optionally substituted heteroaryl."

It should be noted that a chemical moiety that forms part of a larger compound may be described herein using a name commonly accorded it when it exists as a single molecule or a name commonly accorded its radical. For example, the terms "pyridine" and "pyridyl" are accorded the same meaning when used to describe a moiety attached to other chemical moieties. Thus, the two phrases "XOH, wherein X is pyridyl" and "XOH, wherein X is pyridine" are accorded the same meaning, and encompass the compounds pyridin-2-ol, pyridin-3-ol and pyridin-4-ol.

It should also be noted that if the stereochemistry of a structure or a portion of a structure is not indicated with, for example, bold or dashed lines, the structure or the portion of the structure is to be interpreted as encompassing all stereoisomers of it. Moreover, any atom shown in a drawing with unsatisfied valences is assumed to be attached to enough hydrogen atoms to satisfy the valences. In addition, chemical bonds depicted with one solid line parallel to one dashed line encompass both single and double (*e*.*g*., aromatic) bonds, if valences permit.

### 5.2. Compounds

This invention relates to a dual SGLT1/2 inhibitor that is of the formula: 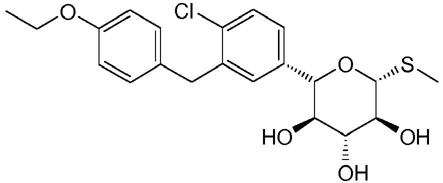 This compound can be prepared by methods known in the art. *See, e.g.,* U.S. patent application publication nos. 20080113922 and 20080221164.

The Applicant has found that this compound has an SGLT1 IC₅₀ : SGLT2 IC₅₀ ratio of about 20. Crystalline solid forms of this compound are described in International Application Publication No. WO 2010/009197, and include anhydrous forms 1 and 2.

Crystalline anhydrous (2S,3R,4R,5S,6R)-2-(4-chloro-3-(4-ethoxybenzyl)phenyl)-6-(methylthio)tetrahydro-2H-pyran-3,4,5-triol Form 1 has a differential scanning calorimetry (DSC) endotherm at about 124°C. In this context, the term "about" means ± 5.0°C. In one embodiment, the form provides an X-ray powder diffraction (XRPD) pattern that contains peaks at one or more of about 4.0, 8.1, 9.8, 14.0 and/or 19.3 degrees 2θ. In this context, the term "about" means ± 0.3 degrees.

Crystalline anhydrous Form 2 has a DSC endotherm at about 134°C. In this context, the term "about" means ± 5.0°C. In one embodiment, the form provides an XRPD pattern that contains peaks at one or more of about 4.4, 4.8, 14.5, 14.7, 15.5, 21.2, 22.1 and/or 23.8 degrees 2θ. In this context, the term "about" means ± 0.3 degrees.

### 5.3. Methods of Use

This invention relates to methods improving the cardiovascular and/or metabolic health of a patient, which comprise administering to a patient in need thereof a safe and efficacious amount of the dual SGLT1/2 inhibitor of the invention.

Patients in need of such improvement include those suffering from diseases or disorders such as atherosclerosis, cardiovascular disease, diabetes (Type 1 and 2), disorders associated with hemoconcentration (*e*.*g*., hemochromatosis, polycythemia vera), hyperglycaemia, hypertension, hypomagnesemia, hyponatremia, lipid disorders, obesity, renal failure (*e*.*g*., stage 1, 2, or 3 renal failure), and Syndrome X. Particular patients suffer from, or are at risk of suffering from, type 2 diabetes mellitus.

The administration of the dual SGLT1/2 inhibitor may effect a decrease in the patient's plasma glucose. The administration may effect an improved oral glucose tolerance in the patient. The administration may lower the patient's post-prandial plasma glucose level. The administration may lower the patient's plasma fructosamine level. The administration may lower the patient's HbA1c level. The administration may reduce the patient's blood pressure (*e*.*g*., systolic and diastolic). The administration may reduce the patient's triglyceride levels.

The patient may be concurrently taking another therapeutic agent. Other therapeutic agents include known therapeutic agents useful in the treatment of the aforementioned disorders including: anti-diabetic agents; anti-hyperglycemic agents; hypolipidemic/lipid lowering agents; anti-obesity agents; anti-hypertensive agents and appetite suppressants.

Examples of suitable anti-diabetic agents include biguanides (*e*.*g*., metformin, phenformin), glucosidase inhibitors (*e*.*g*., acarbose, miglitol), insulins (including insulin secretagogues and insulin sensitizers), meglitinides (*e*.*g*., repaglinide), sulfonylureas (*e*.*g*., glimepiride, glyburide, gliclazide, chlorpropamide, and glipizide), biguanide/glyburide combinations (*e*.*g*., Glucovance), thiazolidinediones (*e*.*g*., troglitazone, rosiglitazone, and pioglitazone), PPAR-alpha agonists, PPAR-gamma agonists, PPAR alpha/gamma dual agonists, glycogen phosphorylase inhibitors, inhibitors of fatty acid binding protein (aP2), glucagon-like peptide-1 (GLP-1) or other agonists of the GLP-1 receptor, and dipeptidyl peptidase IV (DPP4) inhibitors.

Examples of meglitinides include nateglinide (Novartis) and KAD1229 (PF/Kissei).

Examples of thiazolidinediones include Mitsubishi's MCC-555 (disclosed in U.S. Pat. No. 5,594,016), Glaxo-Welcome's GL-262570, englitazone (CP-68722, Pfizer), darglitazone (CP-86325, Pfizer, isaglitazone (MIT/J&J), JTT-501 (JPNT/P&U), L-895645 (Merck), R-119702 (Sankyo/WL), NN-2344 (Dr. Reddy/NN), or YM-440 (Yamanouchi).

Examples of PPAR-alpha agonists, PPAR-gamma agonists and PPAR alpha/gamma dual agonists include muraglitizar, peliglitazar, AR-HO39242 (Astra/Zeneca), GW-409544 (Glaxo-Wellcome), GW-501516 (Glaxo-Wellcome), KRP297 (Kyorin Merck) as well as those disclosed by Murakami et al, Diabetes 47, 1841-1847 (1998), WO 01/21602 and in U.S. Pat. No. 6,653,314.

Examples of aP2 inhibitors include those disclosed in U.S. application Ser. No. 09/391,053, filed Sep. 7, 1999, and in U.S. application Ser. No. 09/519,079, filed Mar. 6, 2000, employing dosages as set out herein.

Examples of DPP4 inhibitors include sitagliptin (Janiuvia®, Merck), vildagliptin (Galvus®, Novartis), saxagliptin (Onglyza®, BMS-477118), linagliptin (BI-1356), dutogliptin (PHX1149T), gemigliptin(LG Life Sciences), alogliptin(SYR-322, Takeda), those disclosed in WO99/38501, WO99/46272, WO99/67279 (PROBIODRUG), WO99/67278 (PROBIODRUG), and WO99/61431 (PROBIODRUG), NVP-DPP728A (1-[[[2-[(5-cyanopyridin-2-yl)amino]ethyl]amino]acetyl]-2-cyano-(S)-pyrro- lidine) (Novartis) as disclosed by Hughes et al, Biochemistry, 38(36), 11597-11603, 1999, TSL-225 (tryptophyl-1,2,3,4-tetrahydroisoquinoline-3-carboxylic acid (disclosed by Yamada et al, Bioorg. & Med. Chem. Lett. 8 (1998) 1537-1540), 2-cyanopyrrolidides and 4-cyanopyrrolidides, as disclosed by Ashworth et al, Bioorg. & Med. Chem. Lett., Vol. 6, No. 22, pp 1163-1166 and 2745-2748 (1996), the compounds disclosed in U.S. application Ser. No. 10/899,641, WO 01/868603 and U.S. Pat. No. 6,395,767, employing dosages as set out in the above references.

Examples of anti-hyperglycemic agents include glucagon-like peptide-1 (GLP-1), GLP-1(1-36) amide, GLP-1(7-36) amide, GLP-1(7-37) (as disclosed in U.S. Pat. No. 5,614,492), exenatide (Amylin/Lilly), LY-315902 (Lilly), liraglutide (NovoNordisk), ZP-10 (Zealand Pharmaceuticals A/S), CJC-1131 (Conjuchem Inc), and the compounds disclosed in WO 03/033671.

Examples of hypolipidemic/lipid lowering agents include MTP inhibitors, HMG CoA reductase inhibitors, squalene synthetase inhibitors, fibric acid derivatives, ACAT inhibitors, lipoxygenase inhibitors, cholesterol absorption inhibitors, Na⁺/bile acid co-transporter inhibitors, up-regulators of LDL receptor activity, bile acid sequestrants, cholesterol ester transfer protein (e.g., CETP inhibitors, such as CP-529414 (Pfizer) and JTT-705 (Akros Pharma)), and nicotinic acid and derivatives thereof.

Examples of MTP inhibitors include those disclosed in U.S. Pat. No. 5,595,872, U.S. Pat. No. 5,739,135, U.S. Pat. No. 5,712,279, U.S. Pat. No. 5,760,246, U.S. Pat. No. 5,827,875, U.S. Pat. No. 5,885,983 and U.S. Pat. No. 5,962,440.

Examples of HMG CoA reductase inhibitors include mevastatin and related compounds, as disclosed in U.S. Pat. No. 3,983,140, lovastatin (mevinolin) and related compounds, as disclosed in U.S. Pat. No. 4,231,938, pravastatin and related compounds, such as disclosed in U.S. Pat. No. 4,346,227, simvastatin and related compounds, as disclosed in U.S. Pat. Nos. 4,448,784 and 4,450,171. Other HMG CoA reductase inhibitors which may be employed herein include, but are not limited to, fluvastatin, disclosed in U.S. Pat. No. 5,354,772, cerivastatin, as disclosed in U.S. Pat. Nos. 5,006,530 and 5,177,080, atorvastatin, as disclosed in U.S. Pat. Nos. 4,681,893, 5,273,995, 5,385,929 and 5,686,104, atavastatin (Nissan/Sankyo's nisvastatin (NK-104)), as disclosed in U.S. Pat. No. 5,011,930, visastatin (Shionogi-Astra/Zeneca (ZD-4522)), as disclosed in U.S. Pat. No. 5,260,440, and related statin compounds disclosed in U.S. Pat. No. 5,753,675, pyrazole analogs of mevalonolactone derivatives, as disclosed in U.S. Pat. No. 4,613,610, indene analogs of mevalonolactone derivatives, as disclosed in PCT application WO 86/03488, 6-[2-(substituted-pyrrol-1-yl)-alkyl)pyran-2-ones and derivatives thereof, as disclosed in U.S. Pat. No. 4,647,576, Searle's SC-45355 (a 3-substituted pentanedioic acid derivative) dichloroacetate, imidazole analogs of mevalonolactone, as disclosed in PCT application WO 86/07054, 3-carboxy-2-hydroxy-propane-phosphonic acid derivatives, as disclosed in French Patent No. 2,596,393, 2,3-disubstituted pyrrole, furan and thiophene derivatives, as disclosed in European Patent Application No. 0221025, naphthyl analogs of mevalonolactone, as disclosed in U.S. Pat. No. 4,686,237, octahydronaphthalenes, such as disclosed in U.S. Pat. No. 4,499,289, keto analogs of mevinolin (lovastatin), as disclosed in European Patent Application No. 0142146 A2, and quinoline and pyridine derivatives, as disclosed in U.S. Pat. Nos. 5,506,219 and 5,691,322.

Examples of hypolipidemic agents include pravastatin, lovastatin, simvastatin, atorvastatin, fluvastatin, cerivastatin, atavastatin, and ZD-4522.

Examples of phosphinic acid compounds useful in inhibiting HMG CoA reductase include those disclosed in GB 2205837.

Examples of squalene synthetase inhibitors include α-phosphono-sulfonates disclosed in U.S. Pat. No. 5,712,396, those disclosed by Biller et al., J. Med. Chem. 1988, Vol. 31, No. 10, pp 1869-1871, including isoprenoid (phosphinyl-methyl)phosphonates, as well as other known squalene synthetase inhibitors, for example, as disclosed in U.S. Pat. Nos. 4,871,721 and 4,924,024 and in Biller, S. A., et al., Current Pharmaceutical Design, 2, 1-40 (1996).

Examples of additional squalene synthetase inhibitors suitable for use herein include the terpenoid pyrophosphates disclosed by P. Ortiz de Montellano et al., J. Med. Chem., 1977, 20, 243-249, the farnesyl diphosphate analog A and presqualene pyrophosphate (PSQ-PP) analogs as disclosed by Corey and Volante, J. Am. Chem. Soc. 1976, 98, 1291-1293, phosphinylphosphonates reported by McClard, R. W. et al., J.A.C.S., 1987, 109, 5544 and cyclopropanes reported by Capson, T. L., PhD dissertation, June, 1987, Dept. Med. Chem. U of Utah, Abstract, Table of Contents, pp 16, 17, 40-43, 48-51, Summary.

Examples of fibric acid derivatives which may be employed in combination with the compound relating to this invention include fenofibrate, gemfibrozil, clofibrate, bezafibrate, ciprofibrate, clinofibrate and the like, probucol, and related compounds, as disclosed in U.S. Pat. No. 3,674,836, probucol and gemfibrozil being preferred, bile acid sequestrants, such as cholestyramine, colestipol and DEAE-Sephadex (Secholex, Policexide), as well as lipostabil (Rhone-Poulenc), Eisai E-5050 (an N-substituted ethanolamine derivative), imanixil (HOE-402), tetrahydrolipstatin (THL), istigmastanylphos-phorylcholine (SPC, Roche), aminocyclodextrin (Tanabe Seiyoku), Ajinomoto AJ-814 (azulene derivative), melinamide (Sumitomo), Sandoz 58-035, American Cyanamid CL-277,082 and CL-283,546 (disubstituted urea derivatives), nicotinic acid, acipimox, acifran, neomycin, p-aminosalicylic acid, aspirin, poly(diallylmethylamine) derivatives, such as disclosed in U.S. Pat. No. 4,759,923, quaternary amine poly(diallyldimethylammonium chloride) and ionenes, such as disclosed in U.S. Pat. No. 4,027,009, and other known serum cholesterol lowering agents.

Examples of ACAT inhibitor that may be employed in combination with the compound relating to this invention include those disclosed in Drugs of the Future 24, 9-15 (1999), (Avasimibe); Nicolosi et al., Atherosclerosis (Shannon, Irel). (1998), 137(1), 77-85; Ghiselli, Giancarlo, Cardiovasc. Drug Rev. (1998), 16(1), 16-30; Smith, C., et al., Bioorg. Med. Chem. Lett. (1996), 6(1), 47-50; Krause et al., Editor(s): Ruffolo, Robert R., Jr.; Hollinger, Mannfred A., Inflammation: Mediators Pathways (1995), 173-98, Publisher: CRC, Boca Raton, Fla.; Sliskovic et al., Curr. Med. Chem. (1994), 1(3), 204-25; Stout et al., Chemtracts: Org. Chem. (1995), 8(6), 359-62, or TS-962 (Taisho Pharmaceutical Co. Ltd).

Examples of hypolipidemic agents include up-regulators of LD2 receptor activity, such as MD-700 (Taisho Pharmaceutical Co. Ltd) and LY295427 (Eli Lilly).

Examples of cholesterol absorption inhibitors include SCH48461 (Schering-Plough), as well as those disclosed in Atherosclerosis 115, 45-63 (1995) and J. Med. Chem. 41, 973 (1998).

Examples of ileal Na⁺/bile acid co-transporter inhibitors include compounds as disclosed in Drugs of the Future, 24, 425-430 (1999).

Examples of lipoxygenase inhibitors include 15-lipoxygenase (15-LO) inhibitors, such as benzimidazole derivatives, as disclosed in WO 97/12615, 15-LO inhibitors, as disclosed in WO 97/12613, isothiazolones, as disclosed in WO 96/38144, and 15-LO inhibitors, as disclosed by Sendobry et al., Brit. J. Pharmacology (1997) 120, 1199-1206, and Cornicelli et al., Current Pharmaceutical Design, 1999, 5, 11-20.

Examples of suitable anti-hypertensive agents for use in combination with the compound relating to this invention include beta adrenergic blockers, calcium channel blockers (L-type and T-type; *e*.*g*., diltiazem, verapamil, nifedipine, amlodipine and mybefradil), diuretics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetamide, triamtrenene, amiloride, spironolactone), renin inhibitors, ACE inhibitors (*e*.*g*., captopril, zofenopril, fosinopril, enalapril, ceranopril, cilazopril, delapril, pentopril, quinapril, ramipril, lisinopril), AT-1 receptor antagonists (*e*.*g*., losartan, irbesartan, valsartan), ET receptor antagonists (e.g., sitaxsentan, atrsentan and compounds disclosed in U.S. Pat. Nos. 5,612,359 and 6,043,265), Dual ET/AII antagonist (*e*.*g*., compounds disclosed in WO 00/01389), neutral endopeptidase (NEP) inhibitors, vasopepsidase inhibitors (dual NEP-ACE inhibitors) (e.g., omapatrilat and gemopatrilat), and nitrates.

Examples anti-obesity agents include beta 3 adrenergic agonists, a lipase inhibitors, serotonin (and dopamine) reuptake inhibitors, thyroid receptor beta drugs, 5HT_{2C} agonists, (such as Arena APD-356); MCHR1 antagonists such as Synaptic SNAP-7941 and Takeda T-226926, melanocortin receptor (MC4R) agonists, melanin-concentrating hormone receptor (MCHR) antagonists (such as Synaptic SNAP-7941 and Takeda T-226926), galanin receptor modulators, orexin antagonists, CCK agonists, NPY1 or NPY5 antagonsist, NPY2 and NPY4 modulators, corticotropin releasing factor agonists, histamine receptor-3 (H3) modulators, 11-beta-HSD-1 inhibitors, adinopectin receptor modulators, monoamine reuptake inhibitors or releasing agents, a ciliary neurotrophic factor (CNTF, such as AXOKINE by Regeneron), BDNF (brain-derived neurotrophic factor), leptin and leptin receptor modulators, cannabinoid-1 receptor antagonists (such as SR-141716 (Sanofi) or SLV-319 (Solvay)), and/or an anorectic agent.

Examples of beta 3 adrenergic agonists include AJ9677 (Takeda/Dainippon), L750355 (Merck), or CP331648 (Pfizer) or other known beta 3 agonists, as disclosed in U.S. Pat. Nos. 5,541,204, 5,770,615, 5,491,134, 5,776,983 and 5,488,064.

Examples of lipase inhibitors include orlistat and ATL-962 (Alizyme).

Examples of serotonin (and dopoamine) reuptake inhibitors (or serotonin receptor agonists) include BVT-933 (Biovitrum), sibutramine, topiramate (Johnson & Johnson) and axokine (Regeneron).

Examples of thyroid receptor beta compounds include thyroid receptor ligands, such as those disclosed in WO97/21993 (U. Cal SF), WO99/00353 (KaroBio) and GB98/284425 (KaroBio).

Examples of monoamine reuptake inhibitors include fenfluramine, dexfenfluramine, fluvoxamine, fluoxetine, paroxetine, sertraline, chlorphentermine, cloforex, clortermine, picilorex, sibutramine, dexamphetamine, phentermine, phenylpropanolamine and mazindol.

Examples of anorectic agents include dexamphetamine, phentermine, phenylpropanolamine, and mazindol.

### 5.4. Pharmaceutical Formulations

This invention relates to pharmaceutical compositions comprising the dual SGLT1/2 inhibitor (2S,3R,4R,5S,6R)-2-(4-chloro-3-(4-ethoxybenzyl)phenyl)-6-(methylthio)tetrahydro-2H-pyran-3,4,5-triol, optionally in combination with one or more second active ingredients, such as those described above in Section 5.3.

Tablets comprising the compound are made using crystalline anhydrous (2S,3R,4R,5S,6R)-2-(4-chloro-3-(4-ethoxybenzyl)phenyl)-6-(methylthio)tetrahydro-2H-pyran-3,4,5-triol, preferably form 1 or 2, described herein.

Single unit dosage forms contain predetermined amounts of active ingredients, and may be prepared by methods of pharmacy well known to those skilled in the art. *See, e.g.,* Remington's Pharmaceutical Sciences, 18th ed. (Mack Publishing, Easton PA: 1990).

Typical oral dosage forms are prepared by combining the active ingredient(s) in an intimate admixture with at least one excipient according to conventional pharmaceutical compounding techniques. Because of their ease of administration, tablets and capsules represent the most advantageous oral dosage unit forms. If desired, tablets can be coated by standard aqueous or nonaqueous techniques. Such dosage forms can be prepared by conventional methods of pharmacy. In general, pharmaceutical compositions and dosage forms are prepared by uniformly and intimately admixing the active ingredients with pharmaceutically acceptable excipients and/or diluents, and then shaping the product into the desired presentation if necessary. Disintegrants may be incorporated in solid dosage forms to facility rapid dissolution. Lubricants may also be incorporated to facilitate the manufacture of dosage forms (*e*.*g*., tablets).

### 6. EXAMPLES

### 6.1. In Vitro Human SGLT2 Inhibition Assay

Human sodium/glucose co-transporter type 2 (SGLT2; accession number P31639; GI:400337) was cloned into plRESpuro2 vector for mammalian expression (construct: HA-SGLT2-pIRESpuro2).

HEK293 cells were transfected with the human HA-SGLT2-pIRESpuro2 vector and the bulk stable cell line was selected in presence of 0.5 µg/ml of puromycin. Human HA-SGLT2 cells were maintained in DMEM media containing 10% FBS, 1% GPS and 0.5 µg/ml of puromycin.

The HEK293 cells expressing the human HA-SGLT2 were seeded in 384 well plates (30,000 cells/well) in DMEM media containing 10% FBS, 1% GPS and 0.5 µg/ml of puromycin, then incubated overnight at 37 C, 5% CO₂. Cells were then washed with uptake buffer (140 mM NaCl, 2 mM KCl, 1 mM CaCl₂, 1 mM MgCl₂, 10 mM HEPES, 5 mM Tris, 1 mg/ml bovine serum albumin (BSA), pH 7.3). Twenty microliters of uptake buffer with or without testing compounds were added to the cells. Then, 20 microliters of uptake buffer containing ¹⁴C-AMG (100 nCi) were added to the cells. The cell plates were incubated at 37°C, 5% CO₂ for 1-2 hours. After washing the cells with uptake buffer, scintillation fluid was added (40 microliters/well) and ¹⁴C-AMG uptake was measured by counting radioactivity using a scintillation coulter (TopCoulter NXT; Packard Instruments).

### 6.2. In Vitro Human SGLT1 Inhibition Assay

Human sodium/glucose co-transporter type 1 (SGLT1; accession number NP_000334; GI: 4507031) was cloned into plRESpuro2 vector for mammalian expression (construct: HA-SGLT1-pIRESpuro2).

HEK293 cells were transfected with the human HA-SGLT1-pIRESpuro2 vector and the bulk stable cell line was selected in presence of 0.5 µg/ml of puromycin. Human HA-SGLT1 cells were maintained in DMEM media containing 10% FBS, 1% GPS and 0.5 µg/ml of puromycin.

The HEK293 cells expressing the human HA-SGLT1 were seeded in 384 well plates (30,000 cells/well) in DMEM media containing 10% FBS, 1% GPS and 0.5 µg/ml of puromycin, then incubated overnight at 37 C, 5% CO₂. Cells were then washed with uptake buffer (140 mM NaCl, 2 mM KCI, 1 mM CaCl₂, 1 mM MgCl₂, 10 mM HEPES, 5 mM Tris, 1 mg/ml bovine serum albumin (BSA), pH 7.3). Twenty microliters of uptake buffer with or without testing compounds were added to the cells. Then, 20 microliters of uptake buffer containing ¹⁴C-AMG (100 nCi) were also added to cells. The cell plates were incubated at 37°C, 5% CO₂ for 1-2 hours. After washing the cells with uptake buffer, scintillation fluid was added (40 microliters/well) and ¹⁴C-AMG uptake was measured by counting radioactivity using a scintillation coulter (TopCoulter NXT; Packard Instruments).

### 6.1. Synthesis of ((3aS,5R,6S,6aS)-6-hydroxy-2,2-dimethyltetrahydrofuro[2,3-d][1,3]dioxol-5-yl)(morpholino)methanone

To a 12L three-necked round bottom flask with mechanical stirrer, rubber septum with temperature probe and gas bubbler was charged L-(-)-xylose (504.40 g, 3.360 mol), acetone (5L, reagent grade) and anhydrous MgSO₄ powder (811.23g, 6.740 mol /2.0 equiv). The suspension was set stirring at ambient and then concentrated H₂SO₄ (50 mL, 0.938 mol / 0.28 equiv) was added. A slow mild exotherm was noticed (temperature rose to 24°C over about 1 hr) and the reaction was allowed to stir at ambient overnight. After 16.25 hours, TLC suggested all L-xylose had been consumed, with the major product being the bis-acetonide along with some (3aS,5S,6R,6aS)-5-(hydroxymethyl)-2,2-dimethyltetrahydrofuro[2,3-d][1,3]dioxol-6-ol. The reaction mixture was filtered and the collected solids were washed twice with acetone (500 mL per wash). The stirring yellow filtrate was neutralized with concentrated NH₄OH solution (39 mL) to pH = 8.7. After stirring for 10 min, the suspended solids were removed by filtration. The filtrate was concentrated to afford crude bis-acetonide intermediate as a yellow oil (725.23 g). The yellow oil was suspended in 2.5 L water stirring in a 5L three-necked round bottom flask with mechanical stirrer, rubber septum with temperature probe and gas bubbler. The pH was adjusted from 9 to 2 with 1N aq. HCl (142 mL) and stirred at room temperature for 6 h until GC showed sufficient conversion of the bis-acetonide intermediate to (3aS,5S,6R,6aS)-5-(hydroxymethyl)-2,2-dimethyltetrahydrofuro[2,3-d][1,3]dioxol-6-ol. The reaction was neutralized by the addition of 50% w/w aq. K₂HPO₄ until pH=7. The solvent was then evaporated and ethyl acetate (1.25L) was added to give a white suspension which was filtered. The filtrate was concentrated in vacuo to afford an orange oil which was dissolved in 1 L methyl tert-butyl ether. This solution had KF 0.23 wt% water and was concentrated to afford (3aS,5S,6R,6aS)-5-(hydroxymethyl)-2,2-dimethyltetrahydrofuro[2,3-d][1,3]dioxol-6-ol as an orange oil (551.23g, 86% yield, 96.7 area% pure by GC). ¹H NMR (400 MHz, DMSO-d₆) δ 1.22 (s, 3 H) 1.37 (s, 3 H) 3.51 (dd, J=11.12, 5.81 Hz, 1 H) 3.61 (dd, J=11.12, 5.05 Hz, 1 H) 3.93 - 4.00 (m, 1 H) 3.96 (s, 1 H) 4.36 (d, J=3.79 Hz, 1 H) 4.86 (br. s., 2 H) 5.79 (d, J=3.54 Hz, 1 H). ¹³C NMR (101MHz, DMSO-d₆) δ 26.48, 27.02, 59.30, 73.88, 81.71, 85.48, 104.69, 110.73.

To a solution of (3aS,5S,6R,6aS)-5-(hydroxymethyl)-2,2-dimethyltetrahydrofuro[2,3-d][1,3]dioxol-6-ol (25.0g, 131 mmol) in acetone (375 mL, 15X) and H₂O (125 mL, 5X) was added NaHCO₃ (33.0g, 3.0 equiv), NaBr (2.8g, 20 mol%) and TEMPO (0.40g, 2 mol%) at 20°C. The mixture was cooled to 0-5°C and solid trichloroisocyanuric acid (TCCA, 30.5 g, 1.0 equiv) was then added in portions. The suspension was stirred at 20°C for 24h. Methanol (20 mL) was added and the mixture was stirred at 20°C for 1h. A white suspension was formed at this point. The mixture was filtered, washed with acetone (50 mL, 2X). The organic solvent was removed under vacuum and the aqueous layer was extracted with EtOAc (300 mL, 12X x3) and the combined organic layers were concentrated to afford an oily mixture with some solid residue. Acetone (125 mL, 5X) was added and the mixture was filtered. The acetone solution was then concentrated to afford the desired acid ((3aS,5R,6S,6aS)-6-hydroxy-2,2-dimethyltetrahydrofuro[2,3-d][1,3]dioxole-5-carboxylic acid) as a yellow solid (21.0g, 79%). ¹H NMR (methanol-d₄), δ 6.00 (d, *J* = 3.2 Hz, 1H), 4.72 d, *J* = 3.2 Hz, 1H), 4.53 (d, *J* = 3.2 Hz, 1H), 4.38 (d, *J* = 3.2 Hz, 1H), 1.44 (s, 3H), 1.32 (s, 3H).

To a solution of (3aS,5R,6S,6aS)-6-hydroxy-2,2-dimethyltetrahydrofuro[2,3-d][1,3]dioxole-5-carboxylic acid (5.0g, 24.5 mmol) in THF (100 mL, 20X) was added TBTU (11.8g, 1.5 equiv), *N*-methylmorpholine (NMM, 4.1 mL, 1.5 equiv) and the mixture was stirred at 20°C for 30 min. Morpholine (3.2 mL, 1.5 equiv) was then added, and the reaction mixture was stirred at 20°C for an additional 6h. The solid was filtered off by filtration and the cake was washed with THF (10 mL, 2X x2). The organic solution was concentrated under vacuum and the residue was purified by silica gel column chromatography (hexanes:EtOAc, from 1:4 to 4:1) to afford 4.3 g of the desired morpholine amide (64%) as a white solid. ¹H NMR (CDCl₃), δ 6.02 (d, *J* = 3.2 Hz, 1H), 5.11 (br s, 1H), 4.62 (d, *J* = 3.2 Hz, 1H), 4.58 (d, *J* = 3.2 Hz, 1H), 3.9-3.5 (m, 8H), 1.51 (s, 3H), 1.35 (s, 3H).

### 6.2. Alternative synthesis of ((3aS,5R,6S,6aS)-6-hydroxy-2,2-dimethyltetrahydrofuro[2,3-d][1,3]dioxol-5-yl)(morpholino)methanone

A solution of the diol (3aS,5S,6R,6aS)-5-(hydroxymethyl)-2,2-dimethyltetrahydrofuro[2,3-d][1,3]dioxol-6-ol in acetonitrile (5.38 kg, 65% w/w, 3.50 kg active, 18.40 mol), acetonitrile (10.5 L) and TEMPO (28.4 g, 1 mol %) were added to a solution of K₂HPO₄ (0.32 kg, 1.84 mol) and KH₂PO₄ (1.25 kg, 9.20 mol) in water (10.5 L). A solution of NaClO₂ (3.12 kg, 80% w/w, 27.6 mole, 1.50 eq) in water (7.0 L) and a solution of K₂HPO₄ (2.89 kg, 0.90 eq) in water (3.0 L) were prepared with cooling. Bleach (3.0L, approximate 6% household grade) was mixed with the K₂HPO₄ solution. Approximately 20% of the NaClO₂ solution (1.6 L) and bleach/K₂HPO₄ solution (400 mL, ∼1 mol %) were added. The remainders of the two solutions were added simultaneously. The reaction mixture turned dark red brown and slow exotherm was observed. The addition rate of the NaClO₂ solution was about 40 mL/min (3-4 h addition) and the addition rate for the bleach/K₂HPO₄ solution was about 10-12 mL/min (10 hr addition) while maintaining the batch at 15-25°C. Additional charges of TEMPO (14.3g, 0.5 mol%) were performed every 5-6 hr until the reaction went to completion (usually two charges are sufficient). Nitrogen sweep of the headspace to a scrubber with aqueous was performed to keep the green-yellowish gas from accumulating in the vessel. The reaction mixture was cooled to < 10°C and quenched with Na₂SO₃ (1.4 kg, 0.6 eq) in three portions over 1 hr. The reaction mixture was then acidified with H₃PO₄ until pH reached 2.0-2.1 (2.5-2.7 L) at 5-15°C. The layers were separated and the aqueous layer was extracted with acetonitrile (10.5 L x 3). The combined organic layer was concentrated under vacuo (-100-120 torr) at < 35°C (28-32°C vapor, 45-50°C bath) to low volume (∼ 6-7 L) and then flushed with acetonitrile (40 L) until KF of the solution reached < 1% when diluted to volume of about 12-15Lwith acetonitrile. Morpholine (1.61 L, 18.4 mol, 1.0 eq)was added over 4-6 h and the slurry was aged overnight under nitrogen. The mixture was cooled to 0-5°C and aged for 3 hours then filtered. The filter cake was washed with acetonitrile (10 L). Drying under flowing nitrogen gave 4.13 kg of the morpholine salt of ((3aS,5R,6S,6aS)-6-hydroxy-2,2-dimethyltetrahydrofuro[2,3-d][1,3]dioxole-5-carboxylic acid as a white solid (92-94% pure based on ¹H NMR with 1,4-dimethoxybenzene as the internal standard), 72-75% yield corrected for purity. ¹H NMR (D₂O) δ 5.96 (d, *J* = 3.6 Hz, 1H), 4.58 (d, *J* = 3.6 Hz, 1H), 4.53 (d, *J* = 3.2 Hz, 1H), 4.30 (d, *J* = 3.2 Hz, 1H), 3.84 (m, 2H), 3.18 (m, 2H), 1.40 (s, 1H), 1.25 (s, 1H). ¹³H NMR (D₂O) δ 174.5, 112.5, 104.6, 84.2, 81.7, 75.0, 63.6, 43.1, 25.6, 25.1.

The morpholine salt of ((3aS,5R,6S,6aS)-6-hydroxy-2,2-dimethyltetrahydrofuro[2,3-d][1,3]dioxole-5-carboxylic acid (7.85 kg, 26.9 mol), morpholine (2.40 L, 27.5 mol) and boric acid (340 g, 5.49 mol, 0.2 eq) were added to toluene (31 L). The resulting slurry was degassed and heated at reflux with a Dean-Stark trap under nitrogen for 12 h and then cooled to room temperature. The mixture was filtered to remove insolubles and the filter cake washed with toluene (5 L). The filtrate was concentrated to about 14 L and flushed with toluene (-80 L) to remove excess morpholine. When final volume reached -12 L, heptane (14 L) was added slowly at 60-70°C. The resulting slurry was cooled gradually to room temperature and aged for 3 h. It was then filtered and washed with heptane (12 L) and dry under nitrogen gave a slightly pink solid (6.26 kg, 97% pure, 98% yield). m.p.: 136°C (DSC). ¹H NMR (CDCl₃), δ 6.02 (d, *J* = 3.2 Hz, 1H), 5.11 (br s, 1H), 4.62 (d, *J* = 3.2 Hz, 1H), 4.58 (d, *J* = 3.2 Hz, 1H), 3.9-3.5 (m, 8H), 1.51 (s, 3H), 1.35 (s, 3H). ¹³C NMR (methanol-d₄) δ 26.84, 27.61, 44.24, 47.45, 68.16, 77.14, 81.14, 86.80, 106.87, 113.68, 169.05.

### 6.3. Synthesis of 1-chloro-2-(4-ethoxybenzyl)-4-iodobenzene

A 2L three-necked round bottom flask with mechanical stirrer, rubber septum with temperature probe and pressure-equalized addition funnel with gas bubbler was charged with 2-chloro-5-iodobenzoic acid (199.41 g, 0.706 mol), dichloromethane (1.2L, KF = 0.003 wt% water) and the suspension was set stirring at ambient temperature. Then N,N-dimethylformamide (0.6 mL, 1.1 mol %) was added followed by oxalyl chloride (63 mL, 0.722 mol, 1.02 equiv) which was added over 11 min. The reaction was allowed to stir at ambient overnight and became a solution. After 18.75hours, additional oxalyl chloride (6 mL, 0.069 mol, 0.10 equiv) was added to consume unreacted starting material. After 2 hours, the reaction mixture was concentrated in vacuo to afford crude 2-chloro-5-iodobenzoyl chloride as a pale yellow foam which will be carried forward to the next step.

A jacketed 2L three-necked round bottom flask with mechanical stirrer, rubber septum with temperature probe and pressure-equalized addition funnel with gas bubbler was charged with aluminum chloride (97.68 g, 0.733 mol, 1.04 equiv), dichloromethane (0.65 L, KF = 0.003 wt% water) and the suspension was set stirring under nitrogen and was cooled to about 6°C. Then ethoxybenzene (90 mL, 0.712 mol, 1.01 equiv) was added over 7 minutes keeping internal temperature below 9°C. The resulting orange solution was diluted with dichloromethane (75mL) and was cooled to -7°C. Then a solution of 2-chloro-5-iodobenzoyl chloride (≤ 0.706 mol) in 350 mL dichloromethane was added over 13 minutes keeping the internal temperature below +3°C. The reaction mixture was warmed slightly and held at +5°C for 2 hours. HPLC analysis suggested the reaction was complete and the reaction was quenched into 450mL pre-cooled (∼5°C) 2N aq. HCl with stirring in a jacketed round bottom flask. This quench was done in portions over 10min with internal temperature remaining below 28°C. The quenched biphasic mixture was stirred at 20°C for 45min and the lower organic phase was washed with 1N aq. HCl (200mL), twice with saturated aq. sodium bicarbonate (200mL per wash), and with saturated aq. sodium chloride (200mL). The washed extract was concentrated on a rotary evaporator to afford crude (2-chloro-5-iodophenyl)(4-ethoxyphenyl)methanone as an off-white solid (268.93g, 99.0 area% by HPLC at 220nm, 1.0 area% regioisomer at 200nm, 98.5 % "as-is" yield).

A jacketed 1 L three-necked round bottom flask with mechanical stirrer, rubber septum with temperature probe and gas bubbler was charged with crude (2-chloro-5-iodophenyl)(4-ethoxyphenyl)methanone (30.13 g, 77.93 mmol), acetonitrile (300mL, KF = 0.004 wt% water) and the suspension was set stirring under nitrogen and was cooled to about 5°C. Then triethylsilane (28mL, 175.30 mmol, 2.25 equiv) was added followed by boron trifluoride-diethyletherate (24mL, 194.46mmol, 2.50 equiv) which was added over about 30 seconds. The reaction was warmed to ambient over 30min and was stirred for 17 hours. The reaction was diluted with methyl *tert*-butyl ether (150mL) followed by saturated aq sodium bicarbonate (150mL) which was added over about 1 minutes. Mild gas evolution was noticed and the biphasic solution was stirred at ambient for 45 minutes. The upper organic phase was washed with saturated aq. sodium bicarbonate (100 mL), and with saturated aq. sodium chloride (50mL). The washed extract was concentrated on a rotary evaporator to about one half of its original volume and was diluted with water (70 mL). Further concentration *in vacuo* at 45°C was done until white prills formed which were allowed to cool to ambient while stirring. After about 30 minutes at ambient, the suspended solids were isolated by filtration, washed with water (30 mL), and were dried *in vacuo* at 45°C. After about 2.5 hours, this afforded 1-chloro-2-(4-ethoxybenzyl)-4-iodobenzene as a slightly waxy white granular powder (28.28 g, 98.2 area % by HPLC at 220nm, 97.4 % "as-is" yield).

### 6.4. Synthesis of (4-chloro-3-(4-ethoxybenzyl)phenyl)((3aS,5R,6S,6aS)-6-hydroxy-2,2-dimethyltetrahydrofuro[2,3-d][1,3]dioxol-5-yl)methanone

To a solution of 1-chloro-2-(4-ethoxybenzyl)-4-iodobenzene (500mg, 1.34 mmol) in THF (5.0 mL) was added i-PrMgCl (2.0M in THF, 1.0 mL, 2.00 mmol) at 0-5°C, and the mixture was stirred for 1.5 h at 0-5°C. A solution of (3aS,5R,6S,6aS)-6-hydroxy-2,2-dimethyltetrahydrofuro[2,3-d][1,3]dioxol-5-yl)(morpholino)methanone (146.5 mg, 0.536 mmol) in THF (1.0 mL) was added dropwise at 0-5°C and the mixture was kept stirring for 1h, warmed to 20°C and stirred at 20°C for 2 hours. The reaction was quenched with saturated aq NH₄Cl, extracted with MTBE, washed with brine. The organic layer was concentrated and the residue was purified by silica gel column chromatography to afford the desired ketone (178 mg, 76%) as a white solid. ¹H NMR (CDCl₃) δ 7.88 (dd, *J* = 8.4, 2.0 Hz, 1H), 7.82 (d, *J* = 2.0 Hz, 1H), 7.50 (d, *J* = 8.4 Hz, 1H), 7.12 (d, *J* = 8.4 Hz, 2H), 6.86 (d, *J* = 8.4 Hz, 2H), 6.07 (d, *J* = 3.2 Hz, 1H), 5.21 (d, *J* = 3.2 Hz, 1H), 4.58 (d, *J* = 3.2 Hz, 1H), 4.56 (d, *J* = 3.2 Hz, 1H), 4.16 (d, *J* = 7.2 Hz, 2H), 4.03 (q, *J* = 7.2 Hz, 2H), 1.54 (s, 3H), 1.42 (t, *J* = 7.2 Hz, 3H), 1.37 (s, 3H).

### 6.5. Alternative synthesis of (4-chloro-3-(4-ethoxybenzyl)phenyl)((3aS,5R,6S,6aS)-6-hydroxy-2,2-dimethyltetrahydrofuro[2,3-d][1,3]dioxol-5-yl)methanone

To a 20 L reactor equipped with a mechanical stirrer, a temperature controller and a nitrogen inlet was charged with the iodide (3.00 kg, 8.05 mol) and THF (8 L, 4X to the morpholinoamide) at room temperature and cooled to -5°C. To the above solution was added dropwise a solution of *i*-PrMgCl in THF (Aldrich 2 M, 4.39 L, 8.82 mol) at -5°C over 3 hours. This Grignard solution was used in the ketone formation below.

To a 50 L reactor equipped with a mechanical stirrer, a temperature controller, and a nitrogen inlet was charged the morpholinoamide (HPLC purity = 97 wt%, 2.01 kg, 7.34 mol) and THF (11 L, 5.5X) at room temperature and stirred for 45 minutes at room temperature and for 15 minutes at 30°C. The homogeneous solution was then cooled to -25°C. To this solution was added a solution of *t*-BuMgCl in THF (Aldrich 1 M, 7.32 L, 7.91 mol) at -25°C over 3 hours. Then the above Grignard solution was added to this solution at -20 over 41 minutes. The resulting solution was further stirred at -20°C before quench. The reaction mixture was added to 10 wt% aqueous NH₄Cl (10 L, 5X) at 0°C with vigorous stirring, and stirred for 30 minutes at 0°C. To this mixture was added slowly 6 N HCl (4 L, 2X) at 0°C to obtain a clear solution and stirred for 30 minutes at 10°C. After phase split, the organic layer was washed with 25 wt% aq NaCl (5 L, 2.5X). Then the organic layer was concentrated to a 3X solution under the conditions (200 mbar, bath temp 50°C). EtOAc (24 L, 12X) was added, and evaporated to a 3X solution under the conditions (150 mbar, bath temp 50°C). After removed solids by a polish filtration, EtOAc (4 L, 2X) was added and concentrated to dryness (150 mbar, bath temp 50°C). The wet cake was then transferred to a 50 L reactor equipped with a mechanical stirrer, a temperature controller and a nitrogen inlet. After EtOAc was added, the suspension was heated at 70°C to obtain a 2.5X homogeneous solution. To the resulting homogeneous solution was added slowly heptane (5 L, 2.5X) at the same temperature. A homogeneous solution was seeded and heptane (15 L, 7.5X) was added slowly to a little cloudy solution at 70°C. After stirred for 0.5 h at 70°C, the suspension was slowly cooled to 60°C and stirred for 1 h at 60°C. The suspension was then slowly cool to room temperature and stirred for 14 h at the same temperature. The crystals were collected and washed with heptane (8 L, 4X), dried under vacuum at 45°C to give the desired ketone as fluffy solids (2.57 kg, 100 wt% by HPLC, purity-adjusted yield: 81%).

### 6.6. Synthesis of (2S,3S,4R,5S,6R)-2-(4-chloro-3-(4-ethoxybenzyl)phenyl)-6-(methylthio)tetrahydro-2H-pyran-3,4,5-triyl triacetate

To a solution of the ketone (4-chloro-3-(4-ethoxybenzyl)phenyl)-((3aS,5R,6S,6aS)-6-hydroxy-2,2-dimethyltetrahydrofuro[2,3-d][1,3]dioxol-5-yl)methanone (114.7 g, 0.265 mol) in MeOH (2 L, 17X) was added CeCl₃·7H₂O (118.5g, 1.2 equiv) and the mixture was stirred at 20°C until all solids were dissolved. The mixture was then cooled to -78°C and NaBH₄ (12.03g, 1.2 equiv) was added in portions so that the temperature of the reaction did not exceed -70°C. The mixture was stirred at -78°C for 1 hour, slowly warmed to 0°C and quenched with saturated aq NH₄Cl (550 mL, 5X). The mixture was concentrated under vacuum to remove MeOH and then extracted with EtOAc (1.1L, 10X x2) and washed with brine (550 mL, 5X). The combined organics were concentrated under vacuum to afford the desired alcohol as a colorless oil (crude, 115g). To this colorless oil was added AcOH (650 mL) and H₂O (450 mL) and the mixture was heated to 100°C and stirred for 15 hours. The mixture was then cooled to room temperature (20°C) and concentrated under vacuum to give a yellow oil (crude, -118 g). To this crude oil was added pyridine (500 mL) and the mixture was cooled to 0°C. Then, Ac₂O (195 mL, -8.0 equiv) was added and the mixture was warmed to 20°C and stirred at 20°C for 2h. The reaction was quenched with H₂O (500 mL) and diluted with EtOAc (1000 mL). The organic layer was separated and concentrated under vacuum to remove EtOAc and pyridine. The residue was diluted with EtOAc (1000 mL) and washed with aq NaHSO₄ (1N, 500 mL, x2) and brine (300 mL). The organic layer was concentrated to afford the desired tetraacetate intermediate as a yellow foam (∼133g).

To a solution of tetraacetate (133 g, 0.237 mol assuming pure) and thiourea (36.1, 2.0 equiv) in dioxane (530 mL, 4X) was added trimethylsilyl trifluoromethanesulfonate (TMSOTf) (64.5 mL, 1.5 equiv) and the reaction mixture was heated to 80°C for 3.5 hours. The mixture was cooled to 20°C and Mel (37 mL, 2.5 equiv) and N,N-diisopropylethylamine (DiPEA) (207 mL, 5.0 equiv) was added and the mixture was stirred at 20°C for 3h. The mixture was then diluted with methyl tertiary-butyl ether (MTBE) (1.3 L, 10X) and washed with H2O (650 mL, 5X x2). The organic layer was separated and concentrated under vacuum to give a yellow solid. To this yellow solid was added MeOH (650 mL, 5X) and the mixture was reslurried at 60°C for 2h and then cooled to 0°C and stirred at 0°C for 1 hour. The mixture was filtered and the cake was washed with MeOH (0°C, 70 mL, x3). The cake was dried under vacuum at 45°C overnight to afford the desired triacetate (2S,3S,4R,5S,6R)-2-(4-chloro-3-(4-ethoxybenzyl)phenyl)-6-(methylthio)tetrahydro-2H-pyran-3,4,5-triyl triacetate (88 g, 60% over 4 steps) as a pale yellow solid. ¹H NMR (CDCl₃) δ 7.37 (d, *J* = 8.0 Hz, 1H), 7.20 (dd, *J* = 8.0, 2.0 Hz, 1H), 7.07 (m, 2H), 6.85 (m, 2H), 5.32 (t, *J* = 9.6 Hz, 1H), 5.20 (t, *J* = 9.6 Hz, 1H), 5.05 (t, *J* = 9.6 Hz, 1H), 4.51 (d, *J* = 9.6 Hz, 1H), 4.38 (d, *J* = 9.6 Hz, 1h), 4.04 (m, 2H), 2.17 (s, 3H), 2.11 (s, 3H), 2.02 (s, 3H), 1.73 (s, 3H), 1.42 (t, *J* = 7.2 Hz, 3H).

### 6.7. Alternative synthesis of (2S,3S,4R,5S,6R)-2-(4-chloro-3-(4-ethoxybenzyl)pheny))-6-(methylthio)tetrahydro-2H-pyran-3,4,5-triyl triacetate

To a 50 L reactor under nitrogen atmosphere, 40 L MeOH was charged, followed with the ketone (2.50 kg, 5.78 mol) and CeCl₃·7H₂O (2.16 kg, 1.0 equiv). Methanol (7.5 L) was added as rinse (totally 47.5 L, 19X). A freshly prepared solution of NaBH₄ (87.5 g, 0.4 equiv) in aqueous 1 N NaOH (250 mL) was added slowly (35 min) at 15-25°C. The mixture was then stirred for 15 min. HPLC analysis of the reaction mixture showed approximately 90:10 diastereomeric ratio. The reaction was quenched with 10 wt% aq NH₄Cl (2.5 L, 1X) and the mixture was concentrated under vacuum to 5X, diluted with water (10 L, 4X) and MTBE (12.5L, 5X). The mixture was cooled to 10°C and 6 N aq HCI was added until the pH of the mixture reached 2.0. Stirring was continued for 10 minutes and the layers were separated. The organic layer was washed with H₂O (5L, 2X). The combined aqueous layer was extracted with MTBE (12.5 L, 5X). The combined organic layers were washed with brine (2.5 L, 1X) and concentrated under vacuum to 3X. MeCN (15 L, 6X) was added. The mixture was concentrated again to 10 L (4X) and any solid residue was removed by a polish filtration. The cake was washed with minimal amount of MeCN.

The organic filtrate was transferred to 50 L reactor, and a pre-prepared 20 mol% aqueous H₂SO₄ solution (61.8 mL 98% concentrated H₂SO₄ and 5 L H₂O) was added. The mixture was heated to 80°C for 2 hours and then cooled to 20°C. The reaction was quenched with a solution of saturated aqueous K₂CO₃ (5 L, 2X) and diluted with MTBE (15 L, 6X). The organic layer was separated, washed with brine (5 L, 2X) and concentrated under vacuum to 5 L (2X). MeCN (12.5 L, 5X) was added and the mixture was concentrated to 7.5 L (3X).

The above MeCN solution of (3S,4R,5R,6S)-6-(4-chloro-3-(4-ethoxybenzyl)phenyl)tetrahydro-2H-pyran-2,3,4,5-tetraol was cooled to 10°C, added with dimethylaminopyridine (17.53 g, 2.5 mol%), followed by slow addition of acetic anhydride (3.23 L, 6.0 equiv) and triethylamine (5 L, 2X, 6.0 equiv) so that the temperature of the mixture was kept below 20°C. The reaction was then warmed to 20°C and stirred for 1 hour and diluted with MTBE (15 L, 6X). The mixture was slowly quenched with water (7.5 L, 3X). The organic layer was separated and washed with saturated aqueous KHCO₃ (5L, 2X), 1 N NaHSO₄ (5 L, 2X), and brine (5 L, 2X) in sequence.

The organic layer was then concentrated under vacuum to 5 L (2X). MeCN (12.5 L, 5X) was added and the solution was concentrated to 7.5 L (3X) (KF = 0.08%). Dioxane (12.5 L, 5X) was added and the solution was concentrated to 7.50 L (3X) (KF = 0.02%). Any residual solid was removed by a polish filtration and the cake was washed with minimal amount of dioxane (500 mL).

To the above filtrate was added thiourea (880 g, 2.0 equiv) and TMSOTf (1.57 L, 1.5 equiv). The reaction mixture was heated to 80°C for 3 hours (>97% conversion). The mixture was cooled to 20°C and methyl iodide (541 mL, 1.5 equiv) and diethylisopropylamine (3.02 L, 3.0 equiv) were added and the mixture was stirred at 20°C for 18 hours. An extra methyl iodide charge (90 mL, 0.25 equiv) was added and the mixture was stirred at 20°C for 1 hours. The mixture was then diluted with MTBE (25 L, 10X) and washed with water (12.5 L, 5X x2). The organic layer was separated and concentrated under vacuum to ∼5 L (2X). MeOH (12.5 L, 5X) was added and the mixture was concentrated to 5X to afford a slurry. The mixture was then heated at 60°C for 1 hour and cooled to 0°C and stirred at 0°C for 1 hour. The mixture was filtered and the cake was washed with MeOH (0°C, 2.5 L, 1X x2, 1.0 L, 0.4X). The cake was dried under vacuum at 45°C overnight to afford the desired triacetate (1.49 kg, 47% over 4 steps) as a pale yellow/off-white solid.

### 6.8. Synthesis of (2S,3R,4R,5S,6R)-2-(4-chloro-3-(4-ethoxybenzyl)phenyl)-6-(methylthio)tetrahydro-2H-pyran-3,4,5-triol

To a slurry of (2S,3S,4R,5S,6R)-2-(4-chloro-3-(4-ethoxybenzyl)phenyl)-6-(methylthio)tetrahydro-2H-pyran-3,4,5-triyl triacetate (90.0 g, 0.164mol) in MeOH (900 mL, 10X) was added NaOMe in MeOH (25 wt%, 18 mL, 0.2X) at 20°C and the mixture was stirred at 20°C for 2 hours until all solids disappeared. The mixture was then concentrated to 300 mL, added to H2O (1L) and stirred for 1 hour. The solid was filtered and washed with H2O (100 mL, x3) and the cake was dried under vacuum at 45°C overnight to afford the desired methyl thiolate (67.0g, 95%). 1H NMR (CDCl3) δ 7.38 (d, J = 8.4 Hz, 1H), 7.22 (m, 2H), 7.11 (d, J = 8.8 Hz, 2H), 6.83 (d, J = 8.8 Hz, 2H), 4.35 (d, J = 9.6 Hz, 1H), 4.15 (d, J = 9.6 Hz, 1H), 4.10-3.95 (m, 3H), 3.64 (t, J = 8.8 Hz, 1H), 3.50 (m, 2H), 3.42 (br s, 1H), 2.95 (br s, 1H), 2.57 (br s, 1H), 2.17 (s, 3H), 1.40 (t, J = 7.2 Hz, 3H).

### 6.9. Preparation of Crystalline Anhydrous (2S,3R,4R,5S,6R)-2-(4-chloro-3-(4-ethoxybenzyl)phenyl)-6-(methylthio)tetrahydro-2H-pyran-3,4,5-triol Form 1

Under slightly positive nitrogen pressure, to a 50 L reactor was charged MeOH (12 L) and the triacetate (1.70 Kg, 3.09 mol). Methanol (5L) was added as a rinse. The slurry was then added NaOMe in MeOH (25 wt%, 340 mL, 0.2X) in 15 minutes at 20°C and the mixture was stirred at 20°C for 2 hours until all solids disappeared. To the mixture was added slowly water (25.5 L, 15X) in 45 minutes with 5 g seeding (DSC 123°C). Solids crashed out and the mixture was stirred at 20°C for 1 hour, cooled to 0°C and stirred for 30 minutes. The solid was filtered and washed with water (1.7 L, 1X, x2) and the cake was dried under vacuum at 45°C overnight to afford the title compound (m.p. ≈ 123 °C by DSC peak; 1.28 Kg, 97.7% yield).

### 6.10. Preparation of Crystalline Anhydrous (2S,3R,4R,5S,6R)-2-(4-chloro-3-(4-ethoxybenzyl)phenyl)-6-(methylthio)tetrahydro-2H-pyran-3,4,5-triol Form 2

Under slightly positive nitrogen pressure, to a 50 L reactor was charged MEK (2-butanone, 4 L) and (2S,3R,4R,5S,6R)-2-(4-chloro-3-(4-ethoxybenzyl)phenyl)-6-(methylthio)tetrahydro-2H-pyran-3,4,5-triol Form 1 (1.49 Kg). MEK (3.45 L) was added as a rinse. The mixture was heated to 80°C and heptane (14.9 L, 10X) was slowly added in 1.5 hours. Solids started to crash out and the mixture was charged heptane (14.9 L, 10X) in 6 h. The mixture was stirred at 80°C for 15 hours. The mixture was cooled to 20°C in 3 hours and stirred at 20°C for 1 hour. The solids were filtered and the cake was washed with MEK/heptane (2.5:7.5, v/v, 1.49 L, 1X x2), dried under nitrogen for 12 hours and under vacuum at 50°C for 24 hours to afford the title compound as a white solid (m.p. ≈ 134 °C by DSC peak; 1.48 Kg, 98% recovery).

### 6.11. Alternative Preparation of Crystalline Anhydrous (2S,3R,4R,5S,6R)-2-(4-chloro-3-(4-ethoxybenzyl)phenyl)-6-(methylthio)tetrahydro-2H-pyran-3,4,5-triol Form 2

To a 250 L reactor was charged the triacetate (10 kg) and methanol (75 kg). Sodium methoxide (1.6 kg, 30% solution) was added with 5 kg methanol rinse. The mixture was stirred at room temperature for at least 2 hours or until the reaction was complete. Charcoal (Darco G-60, 1 kg) was added with 5 kg methanol rinse. This mixture was heated at 40°C for 1 h, cooled to room temperature, and filtered through celite. The cake was washed with methanol (10 kg). Water (100 kg) was added and the mixture was concentrated under vacuum. MTBE (200 kg) and water (50 kg) were added and phases were split. The organic layer was washed with water (100 kg) and concentrated under vacuum. MEK (100 kg) was added and the same about of solvent was distilled under vacuum. This MEK addition and distillation was repeated to dry the solution. Enough MEK was added to produce a solution of (2S,3R,4R,5S,6R)-2-(4-chloro-3-(4-ethoxybenzyl)phenyl)-6-(methylthio)tetrahydro-2H-pyran-3,4,5-triol in 50 L MEK. This solution was polish filtered and heptane (100 L) was added at about 80°C. Form 2 seeds (0.1 kg) were added followed by slow addition of heptane (100 L) as 80°C. Heating was continued for 8 h more at 80°C, cooled to 20°C over at least 3 hours, held at this temperature for at least 2 hours, filtered, and washed with MEK/heptane. The cake was dried at 50°C under vacuum to afford the title compound as a white solid (6.6 kg, 86% yield).

### 6.12. Solid Oral Dosage Form of (2S,3R,4R,5S,6R)-2-(4-chloro-3-(4-ethoxybenzyl)phenyl)-6-(methylthio)tetrahydro-2H-pyran-3,4,5-triol

Tablets comprising the active pharmaceutical ingredient (API), (2S,3R,4R,5S,6R)-2-(4-chloro-3-(4-ethoxybenzyl)phenyl)-6-(methylthio)tetrahydro-2H-pyran-3,4,5-triol, were prepared from a common blend, described below in Table 1, which was blended and roller compacted in the first stage of manufacture.

**Table 1 - Common Blend**

| **Material** | **Percent** | **kg** |
|---|---|---|
| Active Ingredient (API) | 70.107 | 3.856* |
| Croscarmellose sodium, NF | 2.944 | 0.147 |
| Colloidal silicon dioxide, NF | 0.916 | 0.046 |
| Microcrystalline cellulose, NF (Avicel PH 102) | 25.379 | 1.269 |
| Magnesium stearate, NF | 0.654 | 0.033 |
| **TOTAL** | **100.00** | **5.00** |

| | | |
|---|---|---|
| * Includes a 10% overage to account for processing loss during initial milling | | |

The API (crystalline anhydrous (2S,3R,4R,5S,6R)-2-(4-chloro-3-(4-ethoxybenzyl)phenyl)-6-(methylthio)tetrahydro-2H-pyran-3,4,5-triol Form 2) was deagglomerated using a conical mill equipped with a 032R screen. The deagglomerated drug substance was blended with the intragranular excipients croscarmellose sodium, collodial silicon dioxide, and microcrystalline cellulose (Avicel PH 102) for 10 minutes using a V-blender. The intragranular portion of the magnesium stearate was then added to the materials and blended for an additional two minutes. The intragranular blend was then passed through a roller compactor for granulation. The roller compacted ribbons were milled using a conical mill equipped with a 79G screen. The milled granules were then passed through the conical mill a second time using a finer 55R screen in order to achieve the desired granule particle size.

Part of the microcrystalline cellulose (Avicel PH 200) was then passed through a 20 mesh screen and charged into a V-blend. The appropriate quantity of the resulting common intragranulation blend was passed through a 20 mesh screen and charged into the V-blender. The requisite amount of the extragranular excipients croscarmellose sodium, colloidal silicon dioxide, talc, and the remaining microcrystalline cellulose (Avicel PH 200) were passed through a 20 mesh screen and charged into the same V-blender and blended for 10 minutes. The extragranular portion of the magnesium stearate was then added to the V-blender and blended for an additional two minutes. The final blends were compressed into 50 mg and 150 mg tablets. The tablet cores were subsequently coated with an aqueous suspension of Opadry II Clear for an approximate weight gain of 3%. Tables 2 and 3 provide the batch formula for the 50 and 150 mg tablets, respectively.

**Table 2 - Batch Formula for 50 mg Tablets**

| **Material** | **Percent** | **mg/tablet** | **kg** |
|---|---|---|---|
| Common Blend | 28.528 | 71.320 | 1.141 |
| Croscarmellose sodium, NF | 3.660 | 9.151 | 0.146 |
| Colloidal silicon dioxide, NF | 1.000 | 2.500 | 0.030 |
| Microcrystalline cellulose, NF (Avicel PH 200) | 65.260 | 163.150 | 2.610 |
| Talc, USP | 0.738 | 1.846 | 0.040 |
| Magnesium stearate, NF | 0.814 | 2.034 | 0.033 |
| **Total (Core Tablet)** | **100.00** | **250.00** | **4.00** |
| Opadry II Clear 85F19250 | 3.00 | 7.500 | 0.120 |
| **Total (Coated Tablet)** | -- | **257.50** | **4.12** |

| | | | |
|---|---|---|---|
| * 71.320 mg of Common Blend results in 50 mg of API in the final product. | | | |

**Table 3 - Batch Formula for 150 mg Tablets**

| **Material** | **Percent** | **mg/tablet** | **kg** |
|---|---|---|---|
| Common Blend | 76.414 | 213.960* | 3.057 |
| Croscarmellose sodium, NF | 2.250 | 6.300 | 0.090 |
| Colloidal silicon dioxide, NF | 1.000 | 2.800 | 0.012 |
| Microcrystalline cellulose, NF (Avicel PH 200) | 19.536 | 54.700 | 0.781 |
| Talc, USP | 0.300 | 0.840 | 0.040 |
| Magnesium stearate, NF | 0.500 | 1.400 | 0.020 |
| **Total (Core Tablet)** | **100.00** | **280.00** | **4.00** |
| Opadry II Clear 85F19250 | 3.00 | 8.400 | 0.120 |
| **Total (Coated Tablet)** | -- | **288.40** | **4.12** |

| | | | |
|---|---|---|---|
| * 213.960mg of Common Blend results in 150 mg of API in the final product. | | | |

### 6.13. Pharmacology of Liquid Oral Dosage Form of (2S,3R,4R,5S,6R)-2-(4-chloro-3-(4-ethoxybenzyl)phenyl)-6-(methylthio)tetrahydro-2H-pyran-3,4,5-triol

Patients (n = 36) with type 2 diabetes mellitus received one of two oral doses of (2S,3R,4R,5S,6R)-2-(4-chloro-3-(4-ethoxybenzyl)phenyl)-6-(methylthio)tetrahydro-2H-pyran-3,4,5-triol, given as 150 mg or 300 mg once daily, or matching placebo, for 28 days in solution. Preliminary data showed significant and sustained glucosuria over the 28-day dosing period for both dose levels when compared to placebo. Adverse events were generally mild and evenly distributed across all dose groups, including placebo, and no evidence of dose-limiting toxicities was observed.

In this study, patients on metformin were taken off of the drug 16 days prior to day 0, the day dosing first began. As shown in FIG. 1, the plasma glucose levels of patients in the placebo group and in the 150 mg/day and 300 mg/day treatment groups increased during that period. Upon treatment, patients in both treatment groups exhibited a rapid, statistically significant decrease in plasma glucose levels.

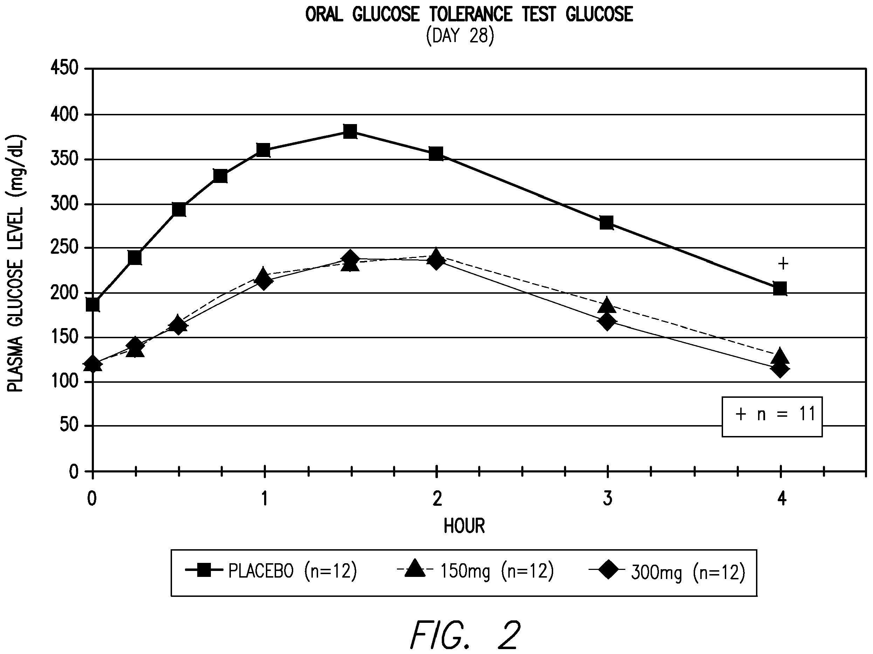

Over the course of the study, the patients' glucose tolerance was tested in a conventional manner. As shown in FIG. 2, patients in both treatment groups exhibited greater glucose tolerance than those in the placebo group.

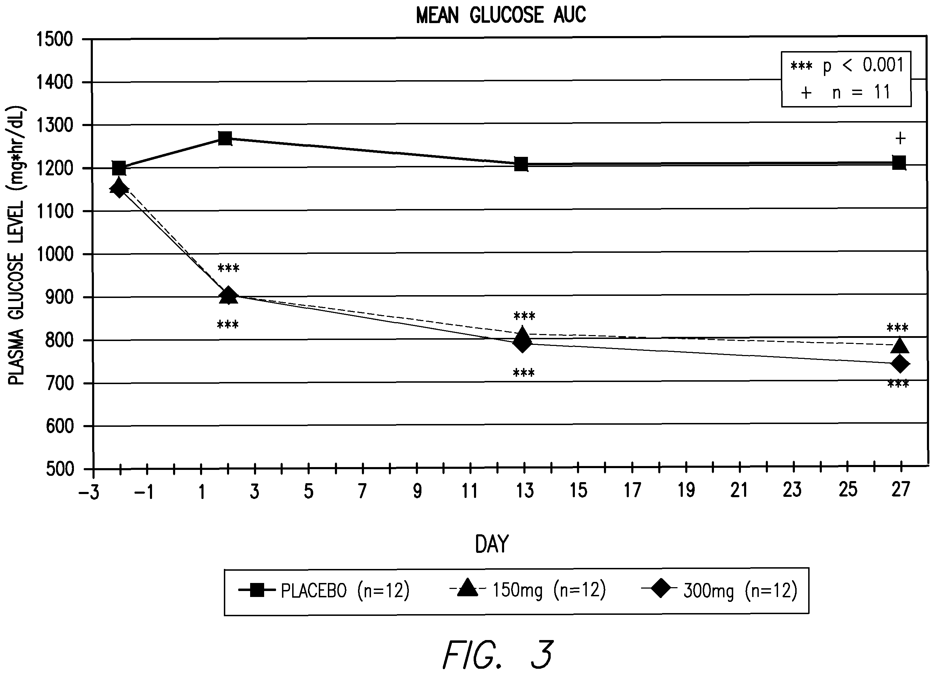

FIG. 3 shows the mean glucose plasma level area under the curve (AUC) of the patients. After just one day of treatment, both the 150 mg/day and 300 mg/day treatment groups exhibited statistically significant decreases in their mean plasma glucose AUCs.

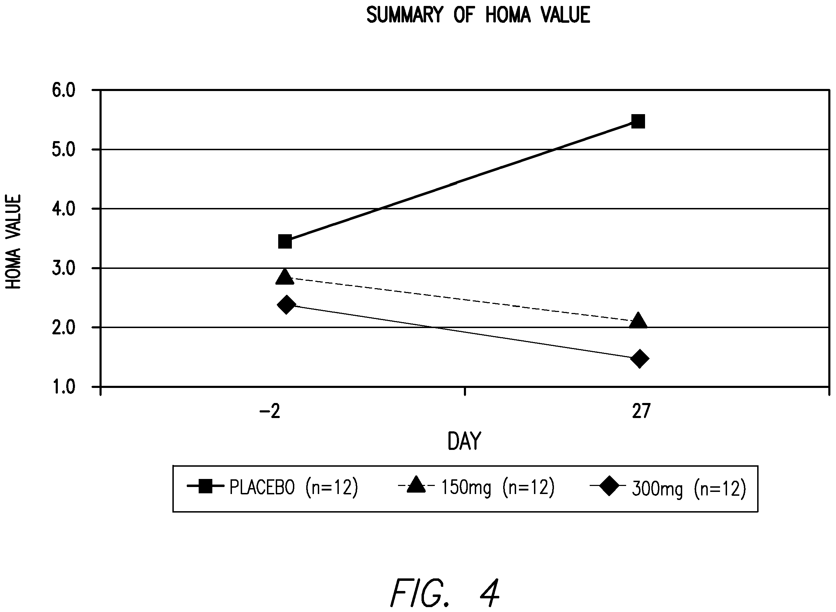

As shown in FIG. 4, patients randomized to the 150 mg/day and 300 mg/day treatment groups showed improved insulin sensitivity compared to placebo. This figure provides a summary of the groups' homeostatic model assessment (HOMA) values.

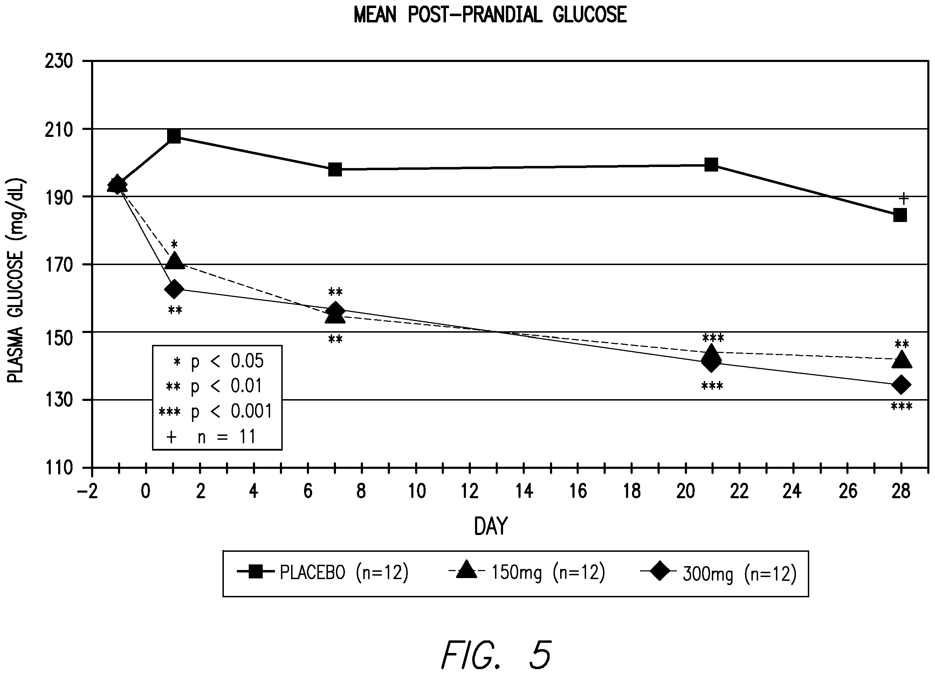

As shown in FIG. 5, patients in both treatment groups exhibited a rapid, statistically significant decrease in post-prandial glucose levels compared to placebo.

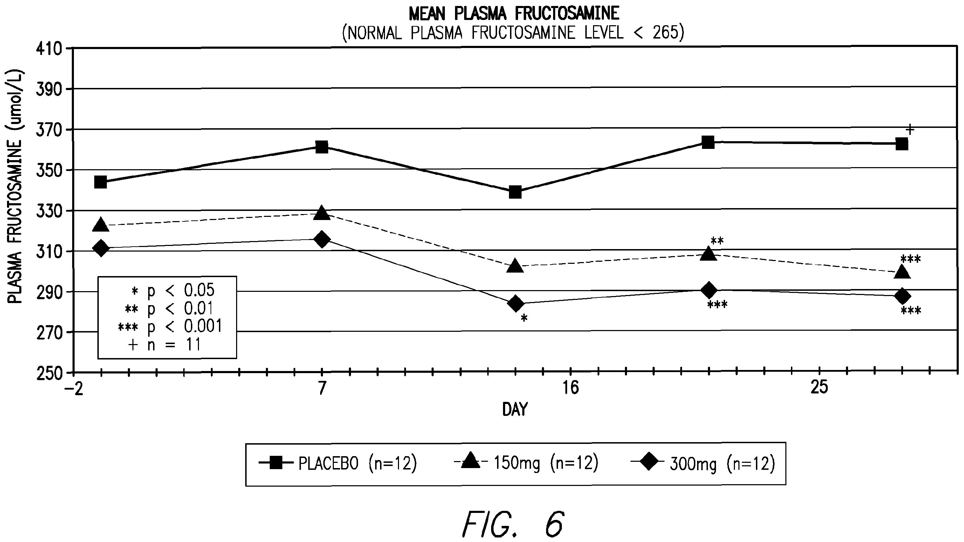

Fructosamine (glycated albumin) is often measured to assess the short-term control of blood sugar. FIG. 6 shows the effect of the compound on patients' mean plasma fructosamine levels.

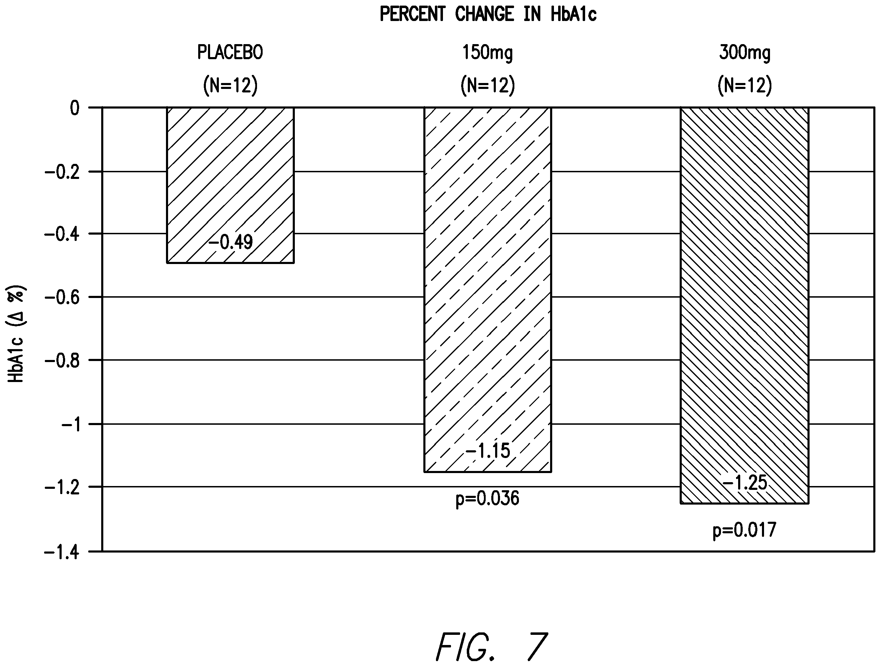

FIG. 7 shows patients' mean percent change in glycated hemoglobin (hemoglobin A1c; HbA1c) levels. HbA1c is a form of hemoglobin used primarily to identify the average plasma glucose concentration over prolonged periods of time. Although this study was only four weeks in duration, patients randomized to the 150 mg/day and 300 mg/day treatment groups exhibited a marked decrease in their mean HbA1c levels.

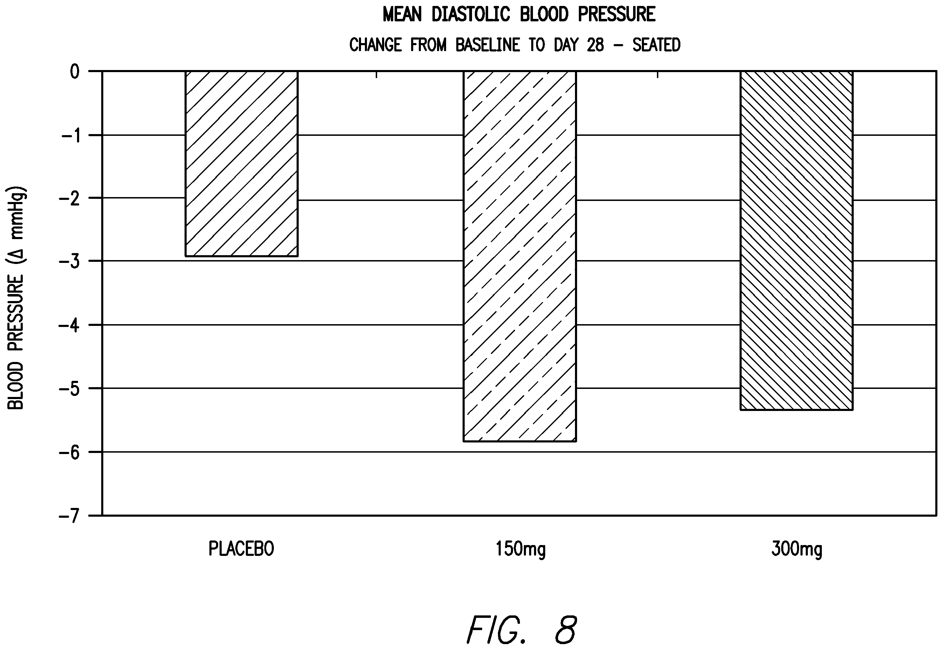

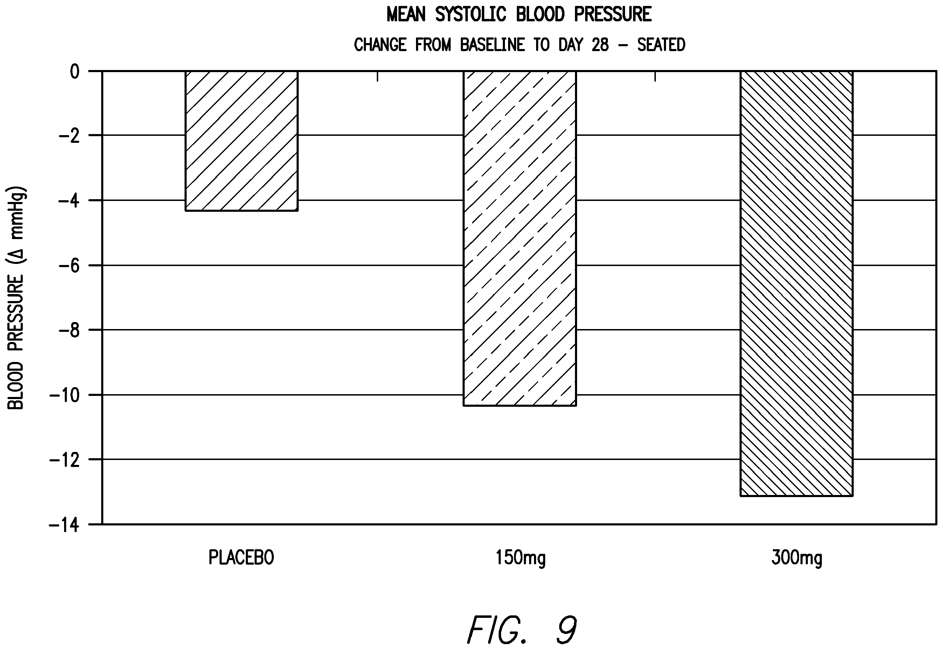

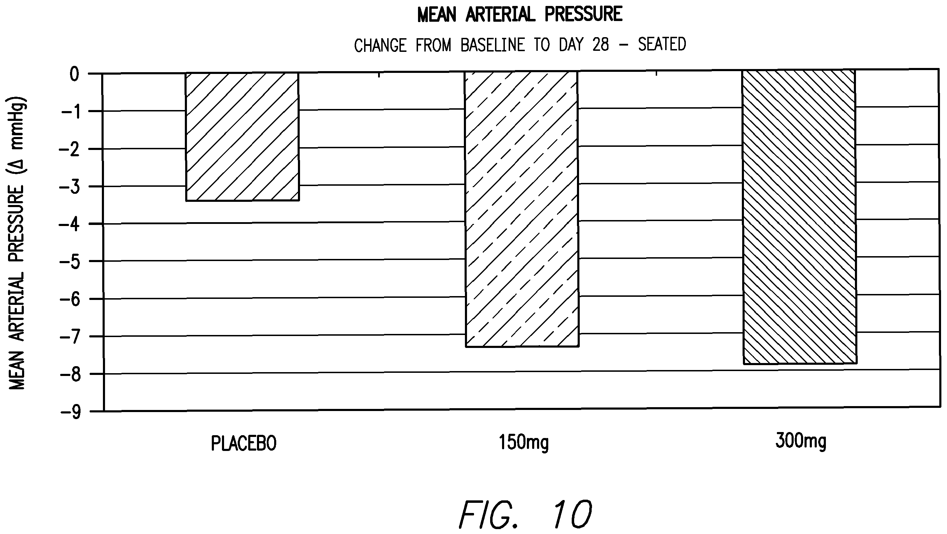

Surprisingly, patients in the 150 mg/day and 300 mg/day treatment groups also exhibited decreased mean diastolic and systolic blood pressures after 28 days of dosing compared to placebo. *See* FIGS. 8 and 9. And as shown in FIG. 10, the mean arterial pressures of patients in both treatment groups also decreased.

As shown below in Table 4, it was found that administration of the compound also lowered patients' serum triglyceride levels and effected weight loss:

**Table 4**

| **Change from Baseline** | **150 mg (n =12)** | **300 mg (n=12)** | **Placebo (n=12)** |
|---|---|---|---|
| Seated Systolic BP (mmHg) | -10.3 | -13.1 | -4.3 |
| Seated Diastolic BP (mmHg) | -5.8 | -5.3 | -2.9 |
| Serum Triglyceride (mg/dL) | -66.6 | -62.8 | -20.2 |
| Change in Weight (%) | -3.4 | -3.7 | -2.2 |

These results demonstrate that within a four-week treatment period, patients receiving the compound exhibited improvements in blood pressure control, weight reduction, and triglyceride levels that were associated with improvements in glycemic parameters.

### 6.14. Pharmacology of Solid Oral Dosage Form of (2S,3R,4R,5S,6R)-2-(4-chloro-3-(4-ethoxybenzyl)phenyl)-6-(methylthio)tetrahydro-2H-pyran-3,4,5-triol

Patients (n = 12) with type 2 diabetes mellitus received one of three oral formulations of 300 mg of (2S,3R,4R,5S,6R)-2-(4-chloro-3-(4-ethoxybenzyl)phenyl)-6-(methylthio)tetrahydro-2H-pyran-3,4,5-triol before breakfast: as two 150 mg tablets, six 50 mg tablets, or 30 mL of 10 mg/mL solution in a randomized sequence implementing a Latin Square crossover design, with a 5-day washout between doses.

The pharmacokinetics of the three formulations were comparable, and adverse events were infrequent. Changes in urinary glucose excretion, fasting plasma glucose (FPG), insulin, postprandial glucose (PPG), peptide YY (PYY), and glucagon-like peptide-1 (GLP-1) were measured at days -1, 1, 6, and 11. As evidenced by the results provided below in Table 5, single doses of the compound markedly improved patients' FPG and PPG levels, which effects were associated with increased GLP-1 and PYY levels.

**Table 5**

| | | **2 x 150 mg** | | **6 x 50 mg** | | **300 mg liquid** | |
|---|---|---|---|---|---|---|---|
| | **Day -1** | **Day of Dosing** | **Change from Day -1** | **Day of Dosing** | **Change from Day -1** | **Day of Dosin 9** | **Change from Day -1** |
| 24-hr urinary glucose (g), mean | 17.3 | 73.1 | 55.8^{a} | 77.5 | 60.3^{a} | 84.8 | 67.5^{a} |
| FPG (mg/dL), mean | 183.0 | 166.0 | -17.0^{b} | 167.17 | -15.8^{b} | 165.0 | -18.0^{b} |
| Insulin (µlU•hr/mL), mean AUC* | 324.9 | 275.5 | -49.4^{a} | 306.9 | -18.0 | 279.9 | -45.0 |
| Total PYY (pmol•hr/L), mean AUC* | 104.3 | 174.72 | 70.4^{b} | 181.9 | 77.59 | 179.3 | 75.0^{b} |
| PPG (mg•hr/dL), mean AUC* | 227.7 | 54.0 | -173.8^{a} | 28.4 | -199.4^{c} | 44.83 | -182.9^{b} |
| Total GLP-1 (pmol•hr/L), mean AUC* | 28.9 | 53.6 | 24.7^{a} | 56.3 | 27.4^{a} | 47.3 | 18.4^{a} |
| Active GLP-1 (pmol•hr/L), mean AUC* | 28.3 | 39.5 | 11.2^{c} | 33.9 | 5.5 | 27.1 | -1.2 |
| ^{a} P<0.001 | | | | | | | |
| ^{b} P<0.05 | | | | | | | |
| ^{c} P<0.01 | | | | | | | |
| * net incremental AUC 0-13 hours (linear trapezoidal rule) | | | | | | | |

Figure 11 further illustrates the effect of the 2 x 150 mg tablet, 6 x 50 mg tablet, and liquid dosage forms on the patients' total GLP-1 levels, wherein the asterisk indicates an area-under-the-curve difference from baseline p value of less than 0.05. The increased levels effected by all three forms is believed to be due to SGLT1 inhibition.

## Claims

1. A tablet comprising an API, croscarmellose sodium, silicon dioxide and microcrystalline cellulose, wherein the active pharmaceutical ingredient ("API") is crystalline anhydrous(2S,3R,4R,5S,6R)-2-(4-chloro-3-(4-ethoxybenzyl)phenyl)-6-(methylthio)tetrahydro-2H-pyran-3,4,5-triol.

2. The tablet of claim 1, wherein the API is crystalline anhydrous (2S,3R,4R,5S,6R)-2-(4-chloro-3-(4-ethoxybenzyl)phenyl)-6-(methylthio)tetrahydro-2H-pyran-3,4,5-triol form 1 having a melting point of 123°C by DSC peak.

3. The tablet of claim 1, wherein the API is crystalline anhydrous (2S,3R,4R,5S,6R)-2-(4-chloro-3-(4-ethoxybenzyl)phenyl)-6-(methylthio)tetrahydro-2H-pyran-3,4,5-triol form 2 having a melting point of 134°C by DSC peak.

4. The tablet of claim 1, wherein the API is present in an amount of 300 mg or less.

5. The tablet of claim 1, which is coated.

6. The tablet of claim 1, which further comprises a second therapeutic agent, which second therapeutic agent is an anti-diabetic agent, anti-hyperglycemic agent, hypolipidemic/lipid lowering agent, anti-obesity agents, anti-hypertensive agent, or appetite suppressant.

7. The tablet of claim 6, wherein the second therapeutic agent is a dipeptidyl peptidase IV ("DPP-4") inhibitor.

8. The tablet of claim 7, wherein the DPP-4 inhibitor is sitagliptin or dutogliptin.

9. A granule comprising an API, croscarmellose sodium, collodial silicon dioxide, and microcrystalline cellulose, wherein the API is (2S,3R,4R,5S,6R)-2-(4-chloro-3-(4-ethoxybenzyl)phenyl)-6-(methylthio)tetrahydro-2H-pyran-3,4,5-triol.

10. The granule of claim 9, wherein the API is crystalline anhydrous (2S,3R,4R,5S,6R)-2-(4-chloro-3-(4-ethoxybenzyl)phenyl)-6-(methylthio)tetrahydro-2H-pyran-3,4,5-triol form 1 having a melting point of 123°C by DSC peak.

11. The granule of claim 9, wherein the API is crystalline anhydrous (2S,3R,4R,5S,6R)-2-(4-chloro-3-(4-ethoxybenzyl)phenyl)-6-(methylthio)tetrahydro-2H-pyran-3,4,5-triol form 2 having a melting point of 134°C by DSC peak.

## Patentansprüche

1. Tablette, umfassend ein API, Croscarmellose-Natrium, Siliciumdioxid und mikrokristalline Cellulose, wobei der aktive pharmazeutischen Wirkstoff ("API") kristallines wasserfreies (2S,3R,4R,5S,6R)-2-(4-Chlor-3-(4-ethoxybenzyl)phenyl)-6-(methylthio)tetrahydro-2H-pyran-3,4,5-triol ist.

2. Tablette nach Anspruch 1, wobei das API kristallines wasserfreies (2S,3R,4R,5S,6R)-2-(4-Chlor-3-(4-ethoxybenzyl)phenyl)-6-(methylthio)tetrahydro-2H-pyran-3,4,5-triol Form 1 mit einem Schmelzpunkt von 123 °C gemäß DSC Peak ist.

3. Tablette nach Anspruch 1, wobei das API kristallines wasserfreies (2S,3R,4R,5S,6R)-2-(4-Chlor-3-(4-ethoxybenzyl)phenyl)-6-(methylthio)tetrahydro-2H-pyran-3,4,5-triol Form 2 mit einem Schmelzpunkt von 134 °C gemäß DSC Peak ist.

4. Tablette nach Anspruch 1, wobei das API in einer Menge von 300 mg oder weniger vorliegt.

5. Tablette nach Anspruch 1, welche beschichtet ist.

6. Tablette nach Anspruch 1, die ferner ein zweites therapeutisches Mittel umfasst, wobei das zweite therapeutische Mittel ein antidiabetisches Mittel, ein antihyperglykämisches Mittel, ein hypolipidämisches / lipidsenkendes Mittel, ein Mittel gegen Fettleibigkeit, ein blutdrucksenkendes Mittel oder ein Appetitzügler ist.

7. Tablette nach Anspruch 6, wobei das zweite therapeutische Mittel ein Dipeptidylpeptidase IV ("DPP-₄") -Inhibitor ist.

8. Tablette nach Anspruch 7, wobei der Dpp₄ Inhibitor Sitagliptin oder Dutogliptin ist.

9. Granulat, umfassend ein API, Croscarmellose-Natrium, kolloidales Siliciumdioxid und mikrokristalline Cellulose, wobei das API (2S, 3R, 4R, 5S, 6R) -2- (4-Chlor-3- (4-ethoxybenzyl) phenyl) -6- (methylthio) tetrahydro-2H-pyran-3,4,5-triol ist.

10. Granulat nach Anspruch 9, wobei das API kristallines wasserfreies (2S, 3R, 4R, 5S, 6R) -2- (4-Chlor-3- (4-ethoxybenzyl) phenyl) -6- (methylthio) tetrahydro-2H-pyran-3,4,5-triol Form 1 mit einem Schmelzpunkt von 123 °C gemäß DSC Peak ist.

11. Granulat nach Anspruch 9, wobei das API kristallines wasserfreies (2S,3R,4R,5S,6R)-2-(4-Chlor-3-(4-ethoxybenzyl)phenyl)-6-(methylthio)tetrahydro-2H-pyran-3,4,5-triol Form 2 mit einem Schmelzpunkt von 134 °C gemäß DSC Peak ist.

## Revendications

1. Un comprimé comprenant un API (ingrédient pharmaceutique actif), croscarmellose sodique, dioxyde de silicone et cellulose microcristalline, dans lequel l'ingrédient pharmaceutique actif (API) est (2S,3R,4R,5S,6R)-2-[4-chloro-3-(4-éthoxybenzyl)phényl)]-6-(méthylthio)-tétrahydro-2H-pyran-3,4,5-triol anhydre cristallin.

2. Le comprimé selon la revendication 1, dans lequel l'API est le (2S,3R,4R,5S,6R)-2-[4-chloro-3-(4-éthoxybenzyl)-phényl)]-6-(méthylthio)-tétrahydro-2H-pyran-3,4,5-triol anhydre cristallin, forme 1, présentant un point de fusion de 123°C au pic DSC.

3. Le comprimé selon la revendication 1, dans lequel l'API est le (2S,3R,4R,5S,6R)-2-[4-chloro-3-(4-éthoxybenzyl)-phényl)]-6-(méthylthio)-tétrahydro-2H-pyran-3,4,5-triol anhydre cristallin, forme 2, présentant un point de fusion de 134°C au pic DSC.

4. Le comprimé selon la revendication 1, dans lequel l'API est présent en une quantité de 300 mg ou moins.

5. Le comprimé selon la revendication 1, qui est revêtu.

6. Le comprimé selon la revendication 1, qui comprend en outre un second agent thérapeutique, lequel second agent thérapeutique est un agent antidiabétique, un agent anti-hyperglycémiant, un agent hypolipidémiant/agent réducteur de lipides hypolipidémiant, des agents anti-obésité, un agent antihypertenseur ou un agent coupe-faim.

7. Le comprimé selon la revendication 6, dans lequel le second agent thérapeutique est un inhibiteur de la dipeptyl peptidase IV (DPP-4).

8. Le comprimé selon la revendication 7, dans lequel l'inhibiteur de la DPP-4 est la sitagliptine ou la dutogliptine.

9. Un granule comprenant un API, de la croscarmellose sodique, du dioxyde de silicium colloïdal et de la cellulose microcristalline, dans lequel l'API est (2S,3R,4R,5S,6R)-2-[4-chloro-3-(4-éthoxybenzyl)-phényl)]-6-(méthylthio)-tétrahydro-2H-pyran-3,4,5-triol.

10. Le granule selon la revendication 9, dans lequel l'API est le (2S,3R,4R,5S,6R)-2-[4-chloro-3-(4-éthoxybenzyl)-phényl)]-6-(méthylthio)-tétrahydro-2H-pyran-3,4,5-triol anhydre cristallin, forme 1, présentant un point de fusion de 123°C au pic DSC.

11. Le granule selon la revendication 9, dans lequel l'API est le (2S,3R,4R,5S,6R)-2-[4-chloro-3-(4-éthoxybenzyl)-phényl)]-6-(méthylthio)tétrahydro-2H-pyran-3,4,5-triol anhydre cristallin, forme 2, présentant un point de fusion de 134°C au pic DSC.
